# EUROPÄISCHE PATENTSCHRIFT

(11) **EP 3 839 576 B1**
(45) Veröffentlichungstag und Bekanntmachung des Hinweises auf die Patenterteilung: **10.09.2025**
(21) Anmeldenummer: 19217562.8
(22) Anmeldetag: 18.12.2019
(51) Int. Cl.: G01T 1/24

(54) **PHOTONENZÄHLENDER RÖNTGENDETEKTOR UND VERFAHREN ZUM BETREIBEN EINES PHOTONENZÄHLENDEN RÖNTGENDETEKTORS**
PHOTON COUNTING X-RAY DETECTOR AND METHOD FOR OPERATING A PHOTON COUNTING X-RAY DETECTOR
DÉTECTEUR DE RAYONS X À COMPTAGE PHOTONIQUE ET PROCÉDÉ DE FONCTIONNEMENT D'UN DÉTECTEUR DE RAYONS X À COMPTAGE PHOTONIQUE

(43) Veröffentlichungstag der Anmeldung: 23.06.2021
(73) Patentinhaber: Siemens Healthineers AG, 91301 Forchheim (DE)
(72) Erfinder: Göderer, Edgar, 91301 Forchheim (DE); Kreisler, Björn, 91353 Hausen (DE); Hupfer, Martin, 91052 Erlangen (DE); Petersilka, Martin, 91325 Adelsdorf (DE)
(74) Vertreter: Siemens Healthineers Patent Attorneys

(56) Entgegenhaltungen:
- CN-A- 103 858 022
- DE-A1- 102012 224 209
- JP-A- 2000 287 104
- US-B2- 8 440 957
- US-B2- 8 772 730

## Beschreibung

Die Erfindung betrifft einen photonenzählenden Röntgendetektor zur Aufnahme eines Röntgenbilddatensatzes eines von einer Röntgenstrahlung durchstrahlten Objektes, ein medizinisches Bildgebungsgerät aufweisend einen photonenzählenden Röntgendetektor und ein Verfahren zum Betreiben eines photonenzählenden Röntgendetektors.

Photonenzählende Röntgendetektoren finden in vielen bildgebenden Anwendungen Einsatz. So werden diese Röntgendetektoren beispielsweise in Computertomographen in der medizinischen Bildgebung genutzt, um ein tomographisches Röntgenbild eines Untersuchungsbereiches eines Patienten zu erzeugen.

Als photonenzählendender Röntgendetektor kann insbesondere ein photonenzählendender, direkt-konvertierender Röntgendetektoren eingesetzt werden. Eintreffende Röntgenstrahlung bzw. Photonen können in solchen Röntgendetektoren durch ein geeignetes Konvertermaterial in elektrische Pulse umgewandelt werden. Als Konvertermaterial können beispielsweise CdTe, CZT, HgI2, GaAs oder andere verwendet werden. Die elektrischen Pulse werden von einer Auswerteelektronik, beispielsweise einem integrierten Schaltkreis (Application Specific Integrated Circuit, ASIC), bewertet. In zählenden Röntgendetektoren wird einfallende Röntgenstrahlung dann durch Zählen der elektrischen Pulse, welche durch die Absorption von Röntgenphotonen im Konvertermaterial ausgelöst werden, gemessen. Die Höhe oder auch die Länge eines erzeugten elektrischen Pulses ist in der Regel außerdem proportional zur Energie des absorbierten Röntgenphotons. Dadurch kann eine spektrale Information durch den Vergleich der Höhe bzw. Länge des elektrischen Pulses mit einer Energieschwelle extrahiert werden. Häufig weisen photonenzählende Röntgendetektor mehrere einstellbare Energieschwellen für einen Vergleich der erzeugten elektrischen Pulse auf, so dass energieaufgelöste Messungen in Abhängigkeit von mehreren durch die Energieschwellen definierten Energiebereiche ermöglicht sind.

Die Nutzung photonenzählender Detektoren in der Röntgenbildgebung bietet eine Reihe von Vorteilen gegenüber energieintegrierenden Detektoren. So ermöglichen sie eine hohe Ortsauflösung und eine intrinsisch energieaufgelöste Messung.

Die Bildqualität heutiger photonenzählender Röntgendetektoren wird jedoch durch die endliche Ausdehnung der erzeugten Ladungswolken (sowie durch die Erzeugung charakteristischer Röntgenstrahlung) im Detektormaterial limitiert. Dies führt dazu, dass nicht immer die gesamte Energie des Röntgenphotons im getroffenen Pixel deponiert wird, sondern ein Teil der Energie in benachbarten Pixeln registriert wird. Als Folge werden zum einen Photonen unter der falschen Energie registriert, zum anderen können auch Photonen in benachbarten Pixeln mehrfach gezählt werden (= Koinzidenz). Diese Koinzidenzen verschlechtern nicht nur die spektralen Eigenschaften des Detektors, sondern führen auch ganz allgemein zu einer Verschlechterung der DQE (detektierbare Quanteneffizienz) des Detektors durch eine Erhöhung des Rauschens und eine Reduzierung der Ortsauflösung. Es handelt sich also um einen Effekt, welcher die Bildqualität für alle Anwendungen degradiert.

Der typische schaltungstechnische Ansatz zur Lösung des Problems besteht in der Implementierung sogenannter "charge summing"-Schaltungen (dt.: Ladungssummations-Schaltungen) in der Auswerteelektronik des Detektors. Hier wird während des Detektionsprozesses im analogen Teil der Auswerteelektronik eines Pixels erkannt, dass Ladung in mehreren benachbarten Pixeln deponiert wurde, und die gesamte Ladung aller Pixel wird einem Pixel (typischerweise dem mit der meisten Ladung oder dem schnellsten Anstieg des Stroms) zugeordnet. Dadurch werden sowohl Doppelzählungen verhindert als auch die ursprüngliche Ladung nahezu wiederhergestellt. Nachteilig an solchen Schaltungen ist, dass die Totzeit der Pixel massiv erhöht wird. Dadurch verschärft sich das Problem des "Pulse-Pile Up" (dt.: Puls-Stapelung), bei dem sich die Signale mehrerer Photonen überlagern und ebenfalls zu verfälschten Messungen führen. Eine gute Hochflussfähigkeit, wie z.B. in der Computertomographie gefordert, ist damit nicht mehr gegeben. Alternativ kann auch durch eine Erhöhung der Pixelgröße (z.B. auf > 0.3 mm Kantenlänge) der Verschlechterung der Energieauflösung und DQE entgegengewirkt werden, allerdings ebenfalls auf Kosten der Hochflussfähigkeit und zusätzlich noch auf Kosten des räumlichen Auflösungsvermögens.

In der DE 10 2011 077 859 B4 ist beispielsweise ein quantenzählender Strahlungsdetektor offenbart mit einem Array von Detektorelementen, die jeweils eine von der Energie auftreffender Strahlungsquanten abhängige Ladungsmenge erzeugen und zur Bildung größerer Detektoreinheiten in Gruppen benachbarter Detektorelemente eingeteilt sind, einer ersten Verarbeitungsstufe, durch die für jede der Gruppen jeweils ein elektrisches Signal bereitgestellt wird, das von der Summe der erzeugten Ladungsmengen der Detektorelemente der Gruppe abhängt, sowie einer zweiten Verarbeitungsstufe, durch die die auf die jeweiligen Gruppen auftreffenden Strahlungsquanten durch Auswertung der bereitgestellten elektrischen Signale gezählt werden, um für jede Gruppe ein Zählergebnis zu erhalten.

In der DE 10 2015 218 585 B4 ist ein zählender Röntgendetektor aufweisend einen Makropixel mit einer Mehrzahl von Subpixeln und aufweisend einen integrierten Schaltkreis offenbart, wobei eine Summationsschaltung zur Bildung eines Summensignals einer ganzzahligen Anzahl an K benachbarten Subpixeln vorgesehen ist, wobei die Eingänge mehrerer erster Diskriminatoren durch einen Schalter mit dem Summensignal verbindbar sind, und die Anzahl K der Subpixel zur Bildung eines Summensignals variabel einstellbar ist.

In "Coincidence counters for charge sharing compensation in spectroscopic photon counting detectors" von Scott S. Hsieh in IEEE Transactions on Medical Imaging. (doi: 10.1109/TMI.2019.2933986) wird außerdem ein Koinzidenz-Zähler ähnlich existierender Zähler basierend auf Energiebereichen vorgeschlagen.

Im Dokument DE 10 2021 224209 A1 ist ein zählender digitaler Röntgendetektor offenbart, welcher eine erste Schaltung und eine zweite Schaltung aufweist, wobei die erste Schaltung dazu ausgebildet ist, das in dem jeweiligen Pixelelement direkt eingegangene Signal einzeln in ein Zählsignal zu wandeln und zu zählen und die zweite Schaltung dazu ausgebildet ist, das in dem jeweiligen Pixelelement direkt eingegangene Signal gemeinsam mit koinzidierend auftretenden Signalen mindestens eines benachbarten Pixelelements in ein Zählsignal zu wandeln und zu zählen, wobei die erste und/oder die zweite Schaltung einzeln und beide gemeinsam aktivierbar sind, und wobei sowohl die erste als auch die zweite Schaltung jeweils mindestens einen Zähler als digitales Speicherelement aufweist.

Im Dokument US 8 772 730 B2 ist ein photonenzählender Detektor offenbart mit NxN Ausleseschaltkreisen, wobei zumindest ein Ausleseschaltkreis eine Vergleichseinheit mit einer Logikschaltung umfassen kann, die so konfiguriert ist, dass sie eine logische Operation unter Verwendung einer Mehrzahl von Vergleichsergebnissen mit einem n-ten Schwellenwert durchführt, wobei die Mehrzahl von Vergleichsergebnissen auf einem Vergleich eines vom Sensor umgewandelten elektrischen Signals mit dem n-ten Schwellenwert in jedem der NxN Ausleseschaltkreisen basiert, und das Ergebnis der logischen Operation an einen Zähler ausgegeben wird.

Im Dokument CN 103 858 022 A ist eine Datenerfassungsvorrichtung für einen Gammastrahlendetektor offenbart umfassend eine Summierschaltung, die zum Empfang mehrerer elektrischer Signale von mehreren Sensoren und zum Summieren der elektrischen Signale zum Erzeugen eines ersten Signals konfiguriert ist und eine Verzögerungssummierschaltung mit mindestens einem Verzögerungselement, die zum selektiven Verzögern und Summieren der elektrischen Signale zum Erzeugen eines zweiten Signals konfiguriert ist.

Im Dokument JP 2000 287104 A ist eine Videosignalkorrektureinheit offenbart, welche einen Addierer und eine Ein-Punkt-Verzögerungseinheit aufweisen kann, um eine kumulative Additionseinheit zu bilden.

Die in der Auswerteelektronik für derartige Schaltungen zur Verfügung stehenden begrenzten Ressourcen (Leistung, Raum, Zeit) erfordern jedoch immer einen gezielten Kompromiss bei der Auswahl umgesetzter Schaltungen und Verfahren, insbesondere wenn auf der anderen Seite gleichzeitig auf andere wünschenswerte Eigenschaften der Auswerteelektronik nicht verzichtet werden soll.

Aufgabe der Erfindung ist es daher einen verbesserten photonenzählenden Röntgendetektor für einen flexiblen Einsatz bereitzustellen.

Die Aufgabe wird gelöst durch die Merkmale der unabhängigen Patentansprüche. Weitere vorteilhafte und teils für sich erfinderische Ausführungsformen und Weiterbildungen der Erfindung sind in den Unteransprüchen und der nachfolgenden Beschreibung dargelegt.

Die Erfindung betrifft einen photonenzählender Röntgendetektor gemäß Anspruch 1.

Der im Rahmen der Erfindung verwendete photonenzählende Röntgendetektor kann auch als zählender oder direkt-konvertierender Röntgendetektor bezeichnet werden. Direkt konvertierende Röntgendetektoren werden meist in einem Stapelaufbau realisiert, bei dem an eine Lage des Konvertermaterials, d.h. an das Konverterelement, unterseitig eine zugeordnete Auswerteeinheit, beispielsweise in Form eines ASICs (Application Specific Integrated Circuit), angebunden ist. Zwischen der Auswerteeinheit und dem Konverterelement kann optional außerdem eine Zwischenschicht, ein Interposer, angeordnet sein, welcher der Stabilität oder auch der Umleitung von Signalleitungen dienen kann. Die Unterseite des Konverterelements weist üblicherweise eine Mehrzahl von Elektroden, im Folgenden auch Sensor-Pixelelektroden genannt, in Form von metallisierten Kontaktelementen matrixförmig auf. Mit diesen ist die Auswerteeinheit signalübertragungstechnisch kontaktiert, üblicherweise verlötet. Üblicherweise steht dabei einem konverterseitigen Kontaktelement jeweils ein pixelförmig ausgeführtes Gegenkontaktelement, im Folgenden auch Pixelelektrode genannt, auf Seiten der Auswerteeinheit entgegen. Die Auswerteeinheit stellt üblicherweise pixelweise Pixelelektroniken für die pixelweise Verarbeitung eines über die Pixelelektroden eingegangenen Signals bereit. Eintreffende Röntgenstrahlung wird im Konvertermaterial des Konverterelements in Abhängigkeit der lokal deponierten Energie eines Röntgenphotons in Ladungsträger konvertiert, basierend auf welchen in der pixelweise Pixelelektronik ein Signal, üblicherweise ein elektrischer Puls, erzeugt wird, welcher üblicherweise pixelweise weiterverarbeitet wird. Ein erfindungsgemäßes Pixelelement kann dabei im Folgenden insbesondere als pixelweise Pixelelektronik, d.h. Elektronik-Pixel, der Auswerteeinheit verstanden werden, welche über die Pixelelektrode signaltechnisch mit dem Konverterelement gekoppelt ist und die vom Konverterelement über die jeweilige Pixelelektrode eingehende Signale weiterverarbeitet. Dem Pixelelement ist ein entsprechendes Detektionsvolumen im Konverterelement zugeordnet, welches durch das elektrische Feld zwischen einer jeweiligen Sensor-Pixelelektrode und einer Top-Elektrode, welche auf der gegenüberliegenden Seite des Konverterelements aufgebracht ist, ausgebildet ist und welches das sensitive Detektionsvolumen eines Pixelelements bildet.

Üblicherweise wird der in einem Pixelelement erzeugte elektrische Puls, dessen Höhe oder auch Länge der deponierten Energie des Röntgenquants im Detektionsvolumen des Pixelelements entspricht, als ein Zählereignis registriert und in eine digitale Speichereinheiten eines Zählelements eingeordnet, d.h. als Pixel-Zählsignal gezählt, wenn der Puls oberhalb eines definierten Schwellwerts, d.h. im Wesentlichen einer Energieschwelle, liegt. Das bedeutet im Wesentlichen, dass in diesem Fall der Zählerstand des dem Schwellwert zugeordneten Zählelements um eine Zähleinheit erhöht wird. Im Falle, dass zwei, drei oder mehr Schwellwerte eingeführt sind, kann das erzeugte elektrische Signal, entsprechend den vordefinierten Schwellwerten, durch ein oder mehrere Zählelemente gezählt werden.

Treten durch Charge-Sharing oder Fluoreszenzen ein Aufteilen der deponierten Energie eines Ereignisses auf zwei oder mehr Pixelelemente auf, können entsprechend in mehr als einem Pixelelement im Wesentlichen gleichzeitig Signale erzeugt werden und damit Mehrfachzählungen, d.h. Koinzidenzen, auftreten. In diesem Fall tritt folglich ein in einem Pixelelement der Vielzahl an Pixelelementen direkt eingegangenes Signal und koinzidierend ein Signal in zumindest einem weiteren Pixelelement der Vielzahl an Pixelelementen auf. Als koinzidierend auftretende Signale gelten dann insbesondere solche, welche innerhalb eines sehr kurzen, gegebenenfalls festlegbaren, Zeitfensters sowohl in einem betrachteten Pixelelement als auch in einem weiteren Pixelelement auftreten. Diese können dann als mit hoher Wahrscheinlichkeit einem einzelnen Photonenereignis zuordenbar gelten.

Der erfindungsgemäße Röntgendetektor weist nun eine Vielzahl an Pixelelementen auf, wobei zumindest eine Teilzahl davon mit einem konfigurierbaren Zähler ausgestattet ist, welcher wahlweise, je nach Konfiguration des Zählers, entweder Pixel-Zählsignale oder Koinzidenz-Zählsignale zählt. Der konfigurierbare Zähler ist also konfigurierbar hinsichtlich eines ersten Zählmodus, in welchem Pixelzählsignale gezählt werden und hinsichtlich eines zweiten Zählmodus, in welchen Koinzidenz-Zählsignale gezählt werden. Der konfigurierbare Zähler kann eine erste und eine zweite Einstellung aufweisen, so dass in einer ersten Einstellung des konfigurierbaren Zählers jeweils das in einem Pixelelement direkt eingegangene Signal als Zählereignis registriert und als Pixel-Zählsignal gezählt wird, und in einer zweiten Einstellung des konfigurierbaren Zählers ein Zählereignis basierend auf dem direkt eingegangenen Signal und einem in zumindest einem weiteren Pixelelement koinzidierend auftretenden Signal registriert und als Koinzidenz-Zählsignal gezählt wird.

Das zumindest eine weitere Pixelelement, auf welchem ein Koinzidenz-Zählsignal basiert, kann dabei bevorzugt zumindest ein direkt benachbartes Pixelelement des jeweiligen Pixelelements umfassen. Es kann jedoch auch ein nicht direkt benachbartes Pixelelement sein.

Bevorzugt ist ein jeweiliges Pixelelement der Teilzahl der Vielzahl an Pixelelementen außerdem ausgebildet mittels des konfigurierbaren Zählers Koinzidenz-Zählsignale zu zählen, welche auf dem in dem jeweiligen Pixelelement der Teilzahl der Vielzahl an Pixelelementen direkt eingegangenen Signal und auf zumindest einem koinzidierend auftretenden Signal mehrerer weiterer Pixelelemente, beispielsweise 2,3,4,8 oder 24, der Vielzahl an Pixelelementen basiert, zu zählen.

Als nicht-beanspruchtes Beispiel, weist der konfigurierbare Zähler insbesondere dazu ein ansteuerbares Schaltelement zur Aktivierung der ersten oder der zweiten Einstellung des konfigurierbaren Zählers auf. Der konfigurierbare Zähler kann dadurch z. B. automatisch angesteuert und entweder die erste oder die zweite Einstellung aktiviert werden. Ebenso kann auch eine manuelle Ansteuerung vorgesehen sein. Die Ansteuerung kann in Verbindung mit einem oder mehreren Parametern erfolgen. Beispielweise kann der Parameter eine Röntgenflussinformation, einen Positionsparameter eines jeweiligen Pixelelements, einen Objektparameter oder einen Anwendungsparameter der medizinischen Anwendung, in deren Rahmen der Röntgenbilddatensatz aufgenommen wird, umfassen. Der zumindest eine konfigurierbare Zähler kann dabei jeweils zeitlich vor oder auch während der Aufnahme des Röntgenbilddatensatzes konfigurierbar sein.

Der Konfigurierbare Zähler umfasst neben einem ansteuerbaren Schaltelement außerdem ein Zählelement. Das Zählelement ist insbesondere ausgebildet, eine Anzahl an Pixel-Zählersignalen oder Koinzidenz-Zählsignalen zu zählen und zumindest zeitweise zu speichern.

Zumindest eine Teilzahl der Vielzahl an Pixelelementen weist zumindest einen konfigurierbaren Zähler auf. Beispielsweise weist lediglich jedes zweite oder dritte Pixelelement in der Matrix an Pixelelementen zumindest einen konfigurierbaren Zähler auf. Es kann aber auch jedes Pixelelement der Vielzahl an Pixelelementen zumindest einen konfigurierbaren Zähler aufweisen. Das heißt, die zumindest eine Teilzahl kann auch die Gesamtheit der Vielzahl an Pixelelementen des Röntgendetektors umfassen, so dass jedes Pixelelement der Vielzahl an Pixelelementen jeweils zumindest einen konfigurierbaren Zähler aufweist.

Die Pixelelemente der Teilzahl der Vielzahl an Pixelelementen können ebenso auch mehr als einen konfigurierbaren Zähler aufweisen, beispielsweise zwei oder drei. Insbesondere können die mehreren konfigurierbaren Zähler jeweils mit einem einstellbaren Schwellwert, d.h. einer Energieschwelle, verknüpft sein. Neben dem zumindest einem konfigurierbaren Zähler kann ein Pixelelement der Teilzahl der Vielzahl an Pixelelementen außerdem auch zumindest einen nicht-konfigurierbaren Zähler aufweisen. Ein nicht-konfigurierbarer Zähler umfasst zumindest ein Zählelement und kann dabei insbesondere ausgebildet sein, ein in einem Pixelelement direkt eingegangenes Signal als Pixel-Zählsignal zu zählen.

Der mittels des photonenzählenden Röntgendetektors aufgenommene Röntgenbilddatensatz kann auf den Pixel-Zählsignalen und/oder den Koinzidenz-Zählsignalen basieren. Die Pixel-Zählsignale und/oder Koinzidenz-Zählsignale können dazu noch weiterverarbeitet werden. Liegen beispielsweise sowohl Pixel-Zählsignale und Koinzidenz-Zählsignale vor können die gezählten Koinzidenz-Zählsignale verwendet werden, um gezählten Pixel-Zählsignale oder darauf basierende Röntgenbildet zu korrigieren und dadurch einen Röntgenbilddatensatz mit einer verbesserten Bildqualität zu ermöglichen.

Vorteilhaft ermöglicht die Erfindung einen flexiblen Einsatz des Röntgendetektors in Abstimmung auf die Notwendigkeiten einer Aufnahme eines Röntgenbilddatensatzes. So kann bei einer geeigneten Konfiguration des zumindest einen konfigurierbaren Zählers der Teilzahl der Vielzahl an Pixelelementen eine Zählung von Zählereignissen ermöglicht werden, zu welchem lediglich diejenigen Röntgenquanten beitragen, welche direkt auf dem Pixelelement (bzw. dem Bereich des Röntgenkonverters, der dem Pixelelement zugeordnet ist) eingetroffen sind. Ebenso kann durch eine geeignete Konfiguration des zumindest einen konfigurierbaren Zählers der Teilzahl der Vielzahl an Pixelelementen eine Zählung ermöglicht werden, bei welcher koinzidierend auf z. B. benachbarten Pixelelementen auftreffende Röntgenquanten mit einbezogen werden. Das heißt, es kann vorteilhaft abgestimmt auf die Anforderungen in Bezug auf die Aufnahme des Röntgenbilddatensatzes lediglich in den Fällen Koinzidenzinformationen gesammelt werden, wenn diese einen positiven Einfluss auf eine resultierende Bildqualität ermöglichen. In anderen Fällen, in welchen eine Koinzidenzinformation keinen oder nur einen geringeren positiven Einfluss hat, kann jedoch auf den konfigurierbaren Zähler als "regulären" Zähler zurückgegriffen werden. Vorteilhaft ist dadurch ein flexibler Einsatz des Röntgendetektors und eine möglichst ressourcenschonende Umsetzung der Pixelelemente ermöglicht. Stehen mehrere konfigurierbare Zähler in einem jeweiligen Pixelelement zur Verfügung kann eine noch flexiblere Anpassung des Röntgendetektors erreicht werden.

Erfindungsgemäß weist der zumindest eine konfigurierbare Zähler einen konfigurierbaren Multiplexer auf, welcher zumindest eine erste und eine zweite Einstellung aufweist, wobei in der ersten Einstellung das Pixel-Zählsignal mittels des konfigurierbaren Zählers gezählt wird, und wobei in der zweiten Einstellung das Koinzidenz-Zählsignal mittels des konfigurierbaren Zählers gezählt wird.

Die Integration eines konfigurierbaren Multiplexers als Schaltelement stellt eine besonders vorteilhafte und einfache Umsetzung des erfindungsgemäßen konfigurierbaren Zählers da. Ein Multiplexer ist im Wesentlichen eine Selektionsschaltung mit der aus einer Anzahl von Eingangssignalen eines ausgewählt und an einen Ausgang des Multiplexers durchgeschaltet werden kann. Der konfigurierbare Multiplexer ist dem Zählelement des konfigurierbaren Zählers vorgeschaltet. Das heißt der konfigurierbare Multiplexer dient als Eingangsmultiplexer des Zählers, wobei in der ersten Einstellung des Multiplexers das basierend auf dem in jedem Pixelelement der Teilzahl der Vielzahl an Pixelelementen direkt eingegangenen Signal als Pixel-Zählsignal mittels des Zählelements gezählt und gespeichert wird und wobei in der zweiten Einstellung des Multiplexers das Koinzidenz-Zählsignal basierend auf dem in dem jeweiligen Pixelelement direkt eingegangenen Signal und auf einem koinzidierend auftretenden Signal mindestens eines weiteren Pixelelements der Vielzahl an Pixelelementen gezählt und gespeichert wird.

Es kann auch vorgesehen sein, dass weitere Eingangssingale am Multiplexer vorgesehen sind, so dass mehr als zwei Einstellungen des konfigurierbaren Zählers konfigurierbar sind. Beispielsweise kann ein Signal basierend auf unterschiedlichen weiterer Pixelelementen jeweils als weiteres Eingangssignal oder auch das Ausgangssignal einer in den Pixelelementen bereitgestellten Summationsschaltung, welche die Signale mehrere Pixelelemente der Vielzahl an Pixelelementen summiert, als Eingangssignal des Multiplexers dienen, wobei je nach Einstellung des Multiplexers eines der Eingangssignale durchgeschalten wird.

Weiterhin kann in einer Ausführungsvariante vorgesehen sein, dass der zumindest eine konfigurierbare Zähler für jedes Pixelelement der Teilzahl der Vielzahl an Pixelelementen individuell und/oder jeweils für eine Gruppe an Pixelelementen der Teilzahl der Vielzahl an Pixelelementen gemeinsam konfigurierbar ist.

Vorteilhaft kann für jedes Pixelelement oder für eine Gruppe an Pixelelementen die für die Bildqualität günstigste Einstellung für die Aufnahme des Röntgenbilddatensatzes einstellbar sein, so dass immer die bestmögliche Bildqualität mittels des Röntgendetektors erzielt werden kann. Vorteilhaft kann zeiteffizient gruppenweise eine gemeinsame Konfiguration ermöglicht sein.

Eine Gruppe kann beispielsweise jeweils einen Makro-Pixel bestehend aus mehreren Pixelelementen definieren. Eine Gruppe an Pixelelementen kann jeweils durch die Position eines jeweiligen Pixelelements relativ zu einem Streustrahlenkollimator oder ähnliches definiert sein. Eine Gruppe kann auch beispielsweise alle randseitig in der Matrix angeordnete Pixelelemente umfassen oder alle mittig in der Matrix angeordnete Pixelelemente umfassen. Randseitig angeordnete Pixelelemente sind insbesondere nicht von allen Seiten eines Pixelelements von benachbarten Pixelelementen umrandet so dass hier gegebenenfalls eine andere Konfiguration des konfigurierbaren Zählers vorteilhaft sein kann im Gegensatz zu mittig angeordneten Pixelelementen. Die Gruppe an Pixelelementen kann auch die gesamte Teilzahl und entsprechend auch die gesamte Vielzahl an Pixelelementen umfassen, wobei zwar eine geringere Flexibilität jedoch eine besonders einfache Umsetzung erreicht werden kann.

Eine vorteilhaft praktische Ausführungsvariante des erfindungsgemäßen photonenzählenden Röntgendetektors umfasst, dass jedes Pixelelement der Vielzahl an Pixelelementen eine mit dem Signaleingang verbundene Wandlungsvorrichtung mit zumindest einem Signalverstärker und eine Anzahl an Komparatoren mit jeweils einem einstellbaren Schwellwert aufweist, und wobei bei jedem Pixelelement der Teilzahl der Vielzahl an Pixelelementen zumindest ein Komparator der Anzahl an Komparatoren mit dem zumindest einen konfigurierbaren Zähler signaltechnisch gekoppelt ist.

Das im Konverterelement in elektrische Ladung umgewandelte und in ein Pixelelement der Auswerteelektronik über die Pixelelektrode eingespeiste Signal wird mit Hilfe des Signalverstärkers verstärkt und dann gezählt, wenn das verstärkte Signal über dem einstellbaren Schwellwert des Komparators liegt. In anderen Worten, liegt das verstärkte Signal über der einstellbaren Energieschwelle des Komparators, wird am Signalausgang des Komparators ein Ausgangssignal bereitgestellt, welches mittels eines damit gekoppelten Zählelement gezählt werden kann. Auf diese Weise werden nur diejenigen Signale, die den Schwellwert überschreiten, so dass Rauschen ausgeblendet werden, gezählt oder nur diejenigen Ereignisse mit Energien oberhalb eines gewünschten Schwellwerts gezählt. Jedes Pixelelement der Vielzahl an Pixelelementen kann eine Mehrzahl an Komparatoren mit jeweils einem einstellbaren Schwellwert aufweisen, wobei das Ausgangssignal eines jeden Komparators mit einem Zählelement, zum Zählen der Pixel-Zählsignale verknüpft sein kann. Auf diese Weise ist eine energieselektive Bildgebung möglich.

Erfindungsgemäß weist dabei zumindest eine Teilzahl der Vielzahl an Pixelelementen zumindest einem konfigurierbaren Zähler auf. Das heißt zumindest ein Komparator jedes Pixelelements der Teilzahl der Vielzahl an Pixelelementen ist signaltechnisch mit einem konfigurierbaren Zähler gekoppelt. Liegt eine Mehrzahl an Komparatoren vor, kann jeder der Komparatoren der Teilzahl der Vielzahl an Pixelelementen mit einem konfigurierbaren Zähler verschaltet sein. Dadurch kann eine größtmögliche Flexibilität erreicht werden Es kann jedoch auch nur ein Teil der Komparatoren eines Pixelelements der Teilzahl der Vielzahl an Pixelelementen signaltechnisch mit einem konfigurierbaren Zähler verknüpft sein. Der Rest der Komparatoren kann mit einem "regulären" Zählelement zum Zählen von Pixel-Zählsignalen, wie oben beschrieben, signaltechnisch verknüpft sein. Dadurch kann der signaltechnische Aufwand und die Leistungsaufnahme reduziert werden.

Gemäß einer weiteren für eine praktische Umsetzung vorteilhaften Ausführungsform des erfindungsgemäßen photonenzählenden Röntgendetektors weist jedes Pixelelement der Teilzahl der Vielzahl an Pixelelementen außerdem zumindest eine Koinzidenzlogik auf, welche mit zumindest einem Komparator des jeweiligen Pixelelements der Teilzahl der Vielzahl an Pixelelementen und mit zumindest einem Komparator des mindestens einen weiteren Pixelelements der Vielzahl an Pixelelementen signaltechnisch gekoppelt ist, wobei das Koinzidenz-Zählsignal auf einem Ausgangssignal der Koinzidenzlogik basiert.

Die Koinzidenzlogik kann auch als digitale Koinzidenzschaltung bezeichnet werden. Die Koinzidenzlogik eines Pixelelement der Teilzahl der Vielzahl an Pixelelementen ist jeweils mit zumindest einem Komparator des jeweiligen Pixelelements der Teilzahl der Vielzahl an Pixelelementen und mit zumindest einem Komparator des mindestens einen weiteren Pixelelements der Vielzahl an Pixelelementen. Das mindestens eine weitere Pixelelement der Vielzahl an Pixelelementen muss dabei nicht notwendigerweise, kann jedoch auch, ein Pixelelement der Teilzahl der Vielzahl an Pixelelementen mit einem konfigurierbaren Zähler sein. Die Koinzidenzlogik kann insbesondere dazu ausgebildet sein, bei Auftreten von koinzidierend auftretenden Signalen in dem betrachteten Pixelelement der Teilzahl der Vielzahl an Pixelelementen und dem zumindest einem weiteren Pixelelement der Vielzahl an Pixelelementen ein Ausgangsignal bereitzustellen, welches mittels eines mit der Koinzidenzlogik signaltechnisch gekoppelten Zählers als Koinzidenz-Zählsignal gezählt werden kann. Das heißt, es kann dabei überprüft werden, ob - ausgehend von einem Pixelelement der Teilzahl der Vielzahl an Pixelelementen - zumindest das eine weitere verknüpfte Pixelelemente ebenfalls ein Zählereignis oberhalb eines gegebenen Schwellwerts detektiert hat. Basierend auf den Ausgangssignalen der mit der Koinzidenzlogik signaltechnisch gekoppelten Komparatoren wird dann bei koinzidierend auftretenden Ereignissen ein Koinzidenzsignal erzeugt. Das Koinzidenz-Zählsignal der Koinzidenzlogik kann dann entsprechend in der zweiten Einstellung des konfigurierbaren Zählers als Pixel-Zählsignal gezählt werden.

Das Ausgangssignal der Koinzidenzlogik eines Pixelelements der Teilzahl der Vielzahl an Pixelelementen kann insbesondere einem ersten Eingangssignal eines konfigurierbaren Multiplexers eines konfigurierbaren Zählers entsprechen. Das Ausgangssignal eines Komparators des Pixelelements der Teilzahl der Vielzahl an Pixelelementen kann insbesondere einem zweiten Eingangssignal des konfigurierbaren Multiplexers entsprechen. Je nach Konfiguration des konfigurierbaren Multiplexers kann entweder das Ausgangssignal des Komparators oder das Ausgangssignal der Koinzidenzlogik als Ausgangsignal des Multiplexers bereitgestellt werden und mittels des mit dem Multiplexer gekoppelten Zählelements gezählt werden.

In einer Variante des erfindungsgemäßen photonenzählenden Röntgendetektors weist jedes Pixelelement der Vielzahl an Pixelelementen eine Mehrzahl an Komparatoren auf, und wobei die zumindest eine Koinzidenzlogik eines Pixelelements der Teilzahl der Vielzahl an Pixelelementen mit mehr als einem Komparator des mindestens einen weiteren Pixelelements der Vielzahl an Pixelelementen signaltechnisch gekoppelt ist.

Beispielsweise können zwei oder mehr Komparatoren des weiteren Pixelelements mit der Koinzidenzlogik eines Pixelelements der Teilzahl der Vielzahl an Pixelelementen signaltechnisch gekoppelt sein. Beispielsweise kann dabei vorgesehen sein, dass auswählbar ist, welches Ausgangssignal der gekoppelten Komparatoren des weiteren Pixelelements zu einem Koinzidenz-Zählsignal im jeweiligen Pixelelement der Teilzahl der Vielzahl beiträgt. In diesem Fall kann vorteilhaft einfach umschaltbar Koinzidenzinformation gegebenenfalls in Abhängigkeit unterschiedlich eingestellter Schwellwerte gesammelt werden.

Beispielweise weist in diesem Fall ein jeweilige Pixelelement der Teilzahl der Vielzahl an Pixelelementen ein weiteres ansteuerbares Schaltelement, beispielsweise einen weiteren Multiplexer, auf, welches mit mehr als einem Komparator des zumindest einen weiteren Pixelelements signaltechnisch gekoppelt ist. Je nach Einstellung des Schaltelements kann dann ein ausgewähltes Ausgangssignal der gekoppelten Komparatoren als Ausgangssignal des Schaltelements bereitgestellt werden und als Grundlage eines Koinzidenz-Zählsignals dienen.

Weiterhin kann in einer Ausführungsvariante der Erfindung vorgesehen sein, dass jedes Pixelelement der Teilzahl der Vielzahl an Pixelelementen zumindest ein Einstellelement zur Laufzeitanpassung oder zur Verzögerung eines Eingangssignals in die zumindest eine Koinzidenzlogik aufweist.

Vorteilhaft können die Ausgangssignale derjenigen Komparatoren des jeweiligen Pixelelements und des zumindest einen weiteren Pixelelements, welche als Eingangssignale der Koinzidenzlogik dienen, aufeinander abgestimmt werden und für die Koinzidenzlogik optimiert werden. Vorteilhaft können unterschiedliche Leitungslängen bei der Zuführung der Signale kompensiert werden. In einer vorteilhaften Ausgestaltung ist jedes der Eingangssignale in die Koinzidenzlogik mit einem solchen Einstellelement verknüpft.

In einer vorteilhaften Ausgestaltung ist das zumindest eine Einstellelemente konfigurierbar, d.h. auch nach Implementierung der Schaltung ansteuerbar und anpassbar. Derart können Unterschiede zwischen den Signaleingängen vorteilhaft auch nachträglich noch ausgeglichen und optimiert werden.

In einer Variante des erfindungsgemäßen photonenzählenden Röntgendetektors weist jedes Pixelelement der Teilzahl der Vielzahl an Pixelelementen mehrere konfigurierbare Zähler auf, welche jeweils mit zumindest einem Komparator des jeweiligen Pixelelements der Teilzahl der Vielzahl an Pixelelementen und mit zumindest einem Komparator des mindestens einen weiteren Pixelelements der Vielzahl an Pixelelementen signaltechnisch gekoppelt ist.

Durch die Bereitstellung mehrerer konfigurierbarer Zähler kann vorteilhaft die Flexibilität der Pixelelemente erhöht werden. Insbesondere können die konfigurierbaren Zähler eines Pixelelements jeweils individuell konfigurierbar ausgebildet sein. Vorteilhat kann je nach medizinischer Anwendung eine Abwägung zwischen der notwendigen spektralen Auflösung und dem Umfang der gesammelten Koinzidenzinformation mittels des Röntgendetektors durchgeführt werden.

Gemäß einer weiteren Ausführungsform des erfindungsgemäßen photonenzählenden Röntgendetektors basiert das Koinzidenz-Zählsignal auf dem in dem jeweiligen Pixelelement der Teilzahl der Vielzahl an Pixelelementen direkt eingegangenem Signal und auf koinzidierend auftretenden Signalen von zwischen einem und 24 weiteren Pixelelementen der Vielzahl an Pixelelementen.

Das heißt, das jeweilige Pixelelement ist ausgebildet ein Koinzidenz-Zählsignal zu bilden und zu zählen, wenn zumindest in einem der ein bis 24 weiteren Pixelelementen ein koinzidierendes Signal auftritt.

Die ein bis 24 weiteren Pixelelemente können bevorzugt in einer matrixartigen Anordnung der Vielzahl an Pixelelementen Teil der direkt benachbarten Pixelelemente, diagonalbenachbarten Pixelelemente oder übernächsten Nachbarn sein.

Bevorzugt umfasst das zumindest eine weitere Pixelelement ein direkt benachbartes Pixelelement des jeweiligen Pixelelements der Teilzahl der Vielzahl an Pixelelementen. Beispielsweise werden Koinzidenz-Zählsignale zweckmäßigerweise in jedem Pixelelement der Vielzahl an Pixelelementen zumindest mit den vier direkt benachbarten weiteren Pixelelementen der Vielzahl an Pixelelementen gebildet. Ein direkt benachbartes Pixelelement eines betrachteten Pixelelements der Teilzahl der Vielzahl an Pixelelementen kann insbesondere einem Pixelelement der Vielzahl an Pixelelementen entsprechen, welches in einem durch die matrixartige Anordnung der Vielzahl an Pixelelementen definierten Pixelraster eine gemeinsame Kante mit dem betrachteten Pixelelement aufweist. Beispielsweise können alternativ oder zusätzlich Koinzidenz-Zählsignale basierend auf einem oder allen diagonal benachbarten Pixelelementen gebildet werden. Vorteilhaft werden zumindest mit denjenigen weiteren Pixelelementen Koinzidenz-Zählsignale gezählt, in welchen mit hoher Wahrscheinlichkeit koinzidierende Signale auftreten. Dies kann zumindest die vier direkt benachbarten Pixelelementen oder die vier direkt benachbarten Pixelelementen gemeinsam mit den diagonal benachbarten Pixelelementen umfassen.

Es kann jedoch auch eine andere Auswahl und/oder Anzahl an weiteren Pixelelementen vorgesehen sein. Es können beispielsweise auch übernächste Nachbarn, d.h. benachbarte Pixelelemente der direkt benachbarten Pixelelemente des betrachteten Pixelelements, hinsichtlich koinzidierend auftretender Signale betrachtet werden. Die Betrachtung und Einbeziehung von übernächsten Nachbarn, kann insbesondere dann vorteilhaft sein, wenn vorgesehen ist die Signale von mehreren Pixelelementen zusammenzufassen, oder bei geringen Pixelgrößen, bei welchen koinzidierend auftretende Signale auch über die durch die Pixelelemente vorgegebenen Abstände hinweg in größerem Ausmaß zu erwarten sind. Jedoch geht mit einer höheren Anzahl an weiteren Pixelelementen und auch eine komplexere Verschaltung der Pixelelemente untereinander einher.

Die Anzahl und Auswahl an weiteren Pixelelementen, auf welchen das Koinzidenz-Zählsignal basiert, kann innerhalb der Teilzahl der Vielzahl an Pixelelementen variieren und/oder konfigurierbar ausgestaltet sein, so dass nach Bereitstellung des Röntgendetektors die Anzahl und Auswahl an weiteren Pixelelementen auswählbar ist.

In einer Ausführungsform des photonenzählenden Röntgendetektor ist die Anzahl und/oder Auswahl an weiteren Pixelelementen der Vielzahl an Pixelelementen, auf welchem das Koinzidenz-Zählsignal basiert für unterschiedliche Pixelelemente unterschiedlich.

Vorteilhaft kann eine besonders flexible Konfiguration des Röntgendetektors ermöglicht werden. Hierbei können vorteilhaft unterschiedliche Randbedingungen der einzelnen Pixelelemente berücksichtig werden. Unterschiedliche Randbedingungen können beispielsweise durch die Anordnung der Pixelelemente relativ zueinander innerhalb einer Pixelmatrix oder durch die Anordnung der Pixelelemente relativ zu einem externen Element, beispielsweise einem Streustrahlengitter oder anderweitigem, vorliegen. So kann vorgesehen sein, dass Pixelelemente, deren Detektionsvolumen beispielsweise teilweise an einer Seite von einem Streustrahlengitter beschattet ist, keine Koinzidenz-Zählsignale basierend auf dem an dieser Seite benachbarten weiteren Pixelelement zählen, da hier durch die Abschattung weniger Beeinträchtigungen durch auftretende Koinzidenzen zu erwarten sein können. Auch kann beispielsweise vorgesehen sein, dass die Vielzahl an Pixelelementen in Makropixel unterteilt ist, welche jeweils einer Gruppe an Pixelelementen entspricht. Beispielsweise werden dann lediglich Koinzidenz-Zählsignale zwischen Pixelelementen innerhalb einer Gruppe gezählt. Je nach Anordnung eines Pixelelement innerhalb der Makropixel-Gruppe kann dann Anzahl und/oder Auswahl an weiteren Pixelelementen vorteilhaft unterschiedlich gewählt sein.

In einer Ausführungsform kann die Anzahl und/oder Auswahl an weiteren Pixelelementen der Vielzahl an Pixelelementen, auf welchem das Koinzidenz-Zählsignal für ein in der Matrix an Pixelelementen randseitig angeordnetes Pixelelement mit weniger benachbarten Pixelelementen basiert, insbesondere verschieden von der Anzahl und/oder Auswahl an weiteren Pixelelementen der Vielzahl an Pixelelementen, auf welchem das Koinzidenz-Zählsignal für ein in der Matrix an Pixelelementen mittig angeordnetes Pixelelement basiert.

Vorteilhaft kann eine besonders flexible Konfiguration des Röntgendetektors ermöglicht werden. Hierbei können vorteilhaft unterschiedliche Randbedingungen der einzelnen Pixelelemente, welche mittig oder randseitig angeordnet sind, berücksichtig werden.

In einer Variante des erfindungsgemäßen photonenzählenden Röntgendetektors umfasst jedes Pixelelement der Teilzahl der Vielzahl an Pixelelementen außerdem zumindest ein elektronisches Element zur Verhinderung einer Paralyse des zumindest einen konfigurierbaren Zählers.

Vorteilhaft kann ein verbessertes Hochflussverhalten der Pixelelemente der Teilzahl der Vielzahl an Pixelelementen erreicht werden. Das elektronisches Element zur Verhinderung der Paralyse kann dabei schaltungstechnisch vor oder auch nach dem konfigurierbaren Schaltelement, d.h. dem Multiplexer, angeordnet sein. Es kann auch zumindest ein elektronisches Element zur Verhinderung der Paralyse für einen jeweiligen Signaleingang einer Koinzidenzlogik vorgesehen sein. Ein solches elektronisches Element zur Verhinderung der Paralyse kann beispielsweise bei dauerhafter Überschreitung einer Komparatorschwelle weitere Zählereignisse induzieren.

Die Erfindung betrifft außerdem ein medizinisches Bildgebungsgerät aufweisend einen erfindungsgemäßen photonenzählenden Röntgendetektor.

Die Merkmale und Vorteile des photonenzählenden Röntgendetektors können dabei direkt auf das medizinischen Bildgebungsgerät übertragen werden.

Das medizinische Bildgebungsgerät kann insbesondere als medizinisches Röntgengerät ausgebildet sein. Das medizinische Bildgebungsgerät kann insbesondere eine dem photonenzählenden Röntgendetektor zugeordnete Röntgenquelle umfassen. Üblicherweise umfasst das medizinische Bildgebungsgerät zumindest einen erfindungsgemäßen photonenzählenden Röntgendetektor und in Gegenüberstellung dazu zumindest eine Röntgenquelle, beispielsweise eine Röntgenröhre. Für die Aufnahme des Röntgenbilddatensatzes kann dann insbesondere zwischen die Röntgenquelle und den photonenzählenden Röntgendetektor das abzubildende Objekt platziert und mittels der Röntgenquelle durchstrahlt werden.

Insbesondere kann das medizinische Bildgebungsgerät als Computertomographie-System ausgebildet sein. Es kann aber auch beispielsweise als C-Bogen-Röntgengerät und/oder Dyna-CT oder anderweitig ausgebildet sein.

Die Erfindung betrifft außerdem ein Verfahren zum Betreiben eines photonenzählenden Röntgendetektors, gemäß Anspruch 14.

Daneben können auch weitere Pixel-Zählsignale und/oder Koinzidenz-Zählsignale basierend auf weiteren konfigurierbaren und/oder regulären, nicht-konfigurierbaren Zählern gezählt werden.

Dabei ist der Röntgendetektor einer Röntgenquelle zur Ausstrahlung einer Röntgenstrahlung zugeordnet.

Beispielsweise kann der Röntgendetektor in einer ersten Konfiguration der Teilzahl der Vielzahl an Pixelelemente bereitgestellt werden, so dass der konfigurierbare Zähler eines jeweiligen Pixelelements der Teilzahl der Vielzahl an Pixelelementen entweder für den ersten Zählmodus oder für den zweiten Zählmodus konfiguriert ist.

Beispielweise kann ein Konfigurieren des photonenzählenden Röntgendetektors durchgeführt werden, wobei zumindest ein Teil der Pixelelemente der Teilzahl der Vielzahl an Pixelelementen der Zählmodus umgeschaltet wird, indem der zumindest eine konfigurierbare Zähler des zumindest eines Teils der Pixelelemente der Teilzahl der Vielzahl an Pixelelementen umgeschaltet wird.

Weiterhin kann eine pixelweise Umwandlung der das Objekt durchstrahlenden und auf dem Röntgendetektor auftreffenden Röntgenstrahlung in elektrische Signale in den Pixelelementen vorgesehen sein. Weiterhin kann einem jeweiligen Pixelelement der Teilzahl der Vielzahl an Pixelelementen eine Umwandlung der elektrischen Signale in Pixel-Zählsignale oder in Koinzidenz-Zählsignale, je nach Konfiguration und Verschaltung der Pixelelemente, und eine Speicherung der gezählten Anzahlen in den Pixelelementen vorgesehen sein. Anschließend kann ein Auslesen der gezählten Anzahlen an Pixel-Zählsignalen und/oder Koinzidenz-Zählsignalen und ein Erstellen eines oder mehrere das Objekt repräsentierender Bilddatensätze vorgesehen sein.

Es kann außerdem vorgesehen sein, dass bei dem erfindungsgemäßen Verfahren das Konfigurieren abhängig von einem oder mehreren Parametern des medizinischen Bildgebungsgeräts dem der erfindungsgemäße Röntgendetektor zugeordnet und/oder mit dem er baulich verbunden ist und/oder der Röntgenanwendung ist. Beispielsweise kann für jedes Pixelelement individuell oder jeweils für Gruppen an Pixelelementen die für die Bildqualität günstigste Variante der Einstellungen konfiguriert werden, so dass immer die bestmögliche Bildqualität erzielt werden kann. Der oder die Parameter können z. B. aus einem Speicher oder einer Systemsteuerung des medizinischen Bildgebungsgeräts abgefragt oder aber direkt ermittelt oder gemessen werden. Beispielsweise umfasst das Verfahren die Abfrage und/oder Ermittlung eines oder mehrerer Parameter.

Das Konfigurieren kann automatisch mittels der Steuereinheit durchgeführt werden. Bei Bedarf kann auch eine manuelle Ansteuerung vorgesehen sein. Die automatische Ansteuerung kann in Verbindung mit dem oder den ermittelten Parametern erfolgen. Dazu kann auch die Abfrage und/oder Ermittlung insbesondere automatisch mittels einer Steuereinheit durchgeführt und für das Konfigurieren angewendet werden.

Nach einer Ausgestaltung der Erfindung wird der Parameter von der Höhe eines Röntgenflusses der Röntgenquelle des Röntgensystems gebildet. In einem solchen Fall kann z. B. vorgesehen sein, dass ab einem gewissen Schwellwert des Röntgenflusses für alle Pixelelemente der Teilzahl der Vielzahl an Pixelelementen die erste Einstellung des zumindest einen konfigurierbaren Zählers eingestellt wird und unterhalb des Schwellwerts die zweite Einstellung.

So können also z. B. bei Ermittlung eines besonders hohen Röntgenflusses (z. B. durch eine Abfrage von der Systemsteuerung oder durch eine Messung), bei denen eine Sammlung von Koinzidenzinformation möglicherweise nicht mehr sinnvoll ist, die konfigurierbaren Zähler lediglich zum Zählen der Pixel-Zählsignale konfiguriert werden. Bei einem mittleren oder geringen Röntgenfluss können dann konfigurierbare Zähler zum Zählen von Koinzidenz-Zählsignalen konfiguriert werden. Es kann auch eine Steuerung z. B. abhängig von der für die Applikation zu erwartenden Zählrate (Dosis/Zeiteinheit) vorgesehen sein.

Auf diese Weise werden bei hohen Röntgenflüssen Fehler durch falsche Koinzidenzen vermieden, bei geringen Röntgenflüssen können hingegen die Koinzidenzen mit in Betracht gezogen werden.

Im Rahmen der Erfindung können Merkmale, welche in Bezug auf unterschiedliche Ausführungsformen der Erfindung und/oder unterschiedliche Anspruchskategorien (Verfahren, Verwendung, Vorrichtung, System, Anordnung usw.) beschrieben sind, zu weiteren Ausführungsformen der Erfindung kombiniert werden. Beispielsweise kann ein Anspruch, der eine Vorrichtung betrifft, auch mit Merkmalen, die im Zusammenhang mit einem Verfahren beschrieben oder beansprucht sind, weitergebildet werden und umgekehrt. Funktionale Merkmale eines Verfahrens können dabei durch entsprechend ausgebildete gegenständliche Komponenten ausgeführt werden. Neben den in dieser Anmeldung ausdrücklich beschriebenen Ausführungsformen der Erfindung sind vielfältige weitere Ausführungsformen der Erfindung denkbar, zu denen der Fachmann gelangen kann, ohne den Bereich der Erfindung zu verlassen, der durch die Ansprüche vorgegeben ist.

Die Verwendung der unbestimmten Artikel "ein" bzw. "eine" schließt nicht aus, dass das betroffene Merkmal auch mehrfach vorhanden sein kann. Die Verwendung des Ausdrucks "aufweisen" schließt nicht aus, dass die mittels des Aus-drucks "aufweisen" verknüpften Begriffe identisch sein können. Beispielsweise weist die medizinische Bildgebungs-vorrichtung die medizinische Bildgebungsvorrichtung auf. Die Verwendung des Ausdrucks "Einheit" schließt nicht aus, dass der Gegenstand, auf den sich der Ausdruck "Einheit" bezieht, mehrere Komponenten aufweisen kann, die räumlich voneinander separiert sind.

Der Ausdruck "basierend auf" kann im Kontext der vorliegenden Anmeldung insbesondere im Sinne des Ausdrucks "unter Verwendung von" verstanden werden. Insbesondere schließt eine Formulierung, der zufolge ein erstes Merkmal basierend auf einem zweiten Merkmal erzeugt (alternativ: ermittelt, bestimmt etc.) wird, nicht aus, dass das erste Merkmal basierend auf einem dritten Merkmal erzeugt (alternativ: ermittelt, bestimmt etc.) werden kann.

Im Folgenden wird die Erfindung anhand von beispielhaften Ausführungsformen unter Hinweis auf die beigefügten Figuren erläutert. Die Darstellung in den Figuren ist schema-tisch, stark vereinfacht und nicht zwingend maßstabsgetreu. Es zeigen:
Fig. 1 ein beispielhaftes Detektormodul mit einem photonenzählenden Röntgendetektor,
Fig. 2 ein Schema zur Veranschaulichung einer signaltechnischen Verschaltung eines Pixelelements gemäß einer ersten Variante,
Fig. 3 ein Schema zur Veranschaulichung einer signaltechnischen Verschaltung eines Pixelelements gemäß einer zweiten Variante,
Fig. 4 ein Schema zur Veranschaulichung einer signaltechnischen Verschaltung eines Pixelelements gemäß einer dritten Variante,
Fig. 5 ein beispielhaftes medizinischen Bildgebungsgerät, und
Fig. 6 einen schematischen Verfahrensablauf eines Verfahrens zum Betreiben eines photonenzählenden Röntgendetektors.

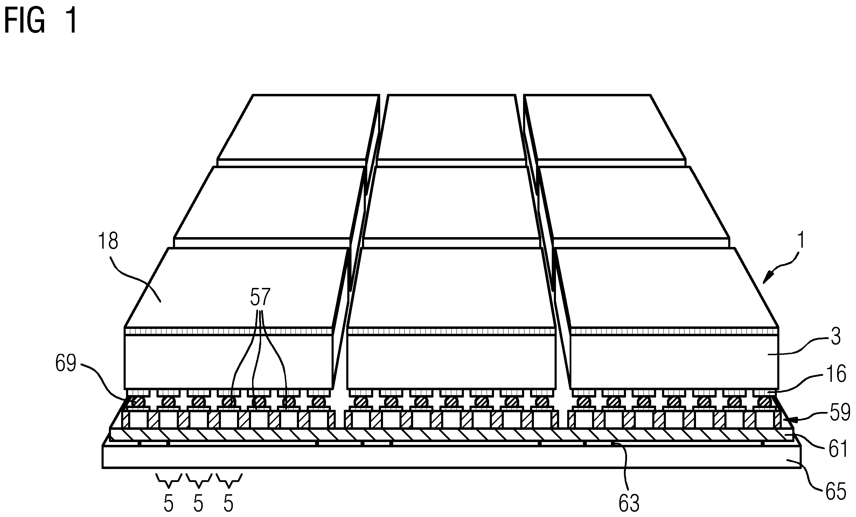

Die Fig. 1 zeigt eine beispielhafte Ausführungsform eines Detektormoduls 51 mit mehreren erfindungsgemäßen Röntgendetektoren 1. In einer bevorzugten Ausführungsform weist das Detektormodul 51 eine zweidimensionale Matrix oder Anordnung einer Mehrzahl von Röntgendetektoren 1 auf. Ein jeweiliger Röntgendetektor 1 weist wiederrum eine Vielzahl an Pixelelementen 5 in einer matrixartigen Anordnung auf, so dass ortsaufgelöste Messungen bereitgestellt werden können.

Ein jeweiliger Röntgendetektor 1 in dem gezeigten Beispiel weist ein Konverterelement 3 auf. Das Konverterelement 3 kann als flächenhafter Direktkonverter, beispielsweise aufweisend CdTe, CZT, CdZnTeSe, CdTeSe, CdMnTe, InP, TlBr2, HgI2, GaAs, Si oder andere als Konvertermaterial, ausgebildet sein. Die Oberseite des Konverterelements 3 weist eine erste Elektrode 18 (Top-Elektrode) auf. Die Unterseite des Konverterelements 3 weist Sensor-Pixelelektroden 16 auf. Die Sensor-Pixelelektroden 16 sind über die elektrisch leitende Verbindungen 69 und die Pixelelektroden 57 mit den Pixelelementen 5 in der Auswerteeinheit 59 verbunden. Die Auswerteeinheit kann beispielsweise in Form eines ASICS ausgebildet sein. Die elektrisch leitenden Verbindungen 69 können beispielsweise als Lotkugeln (bump bonds) oder Lotmaterial in Verbindung mit Kupfersäulen (copper pillars) oder auch anderweitig ausgebildet sein. Die gemeinsame Anzahl der Sensor-Pixelelektroden 16, die Anzahl der leitenden Verbindungen 69, die Anzahl der Pixelelektroden 57 und die Anzahl der Pixelelemente 5 in der Auswerteeinheit 59 sind in der Regel gleich. Ein elektrisches Feld zwischen der ersten Elektrode 18 und einer Sensor-Pixelelektrode 16 bestimmt ein einem Pixelelement 5 zugeordnetes sensitives Detektionsvolumen im Konverterelement 3, welches insbesondere jeweils durch ein elektrisches Feld zwischen den Sensor-Pixelelektroden 16 und der Top-Elektrode 18 ausgebildet ist.

Die Auswerteeinheit 59 ist in dem gezeigten Beispiel auf einem Substrat 61 angeordnet und mit peripherer Elektronik 65, beispielsweise über TSV-Verbindungen 63 ("Through Silicon Via"-Verbindungen) durch das Substrat 61 hindurch, mit einer peripheren Elektronik 65 verbunden ist.

Darüber hinaus kann der Röntgendetektor 1 oder das Röntgendetektormodul 51 auch noch weitere, hier nicht gezeigte Komponenten umfassen.

Üblicherweise wird der in einem Pixelelement 5 erzeugte elektrische Puls, dessen Höhe oder auch Länge der deponierten Energie des Röntgenquants im jeweiligen Detektionsvolumen des Pixelelements 5 entspricht, als ein Zählereignis registriert und in eine digitale Speichereinheiten eines Zählelement 13 eingeordnet, d.h. als Pixel-Zählsignal registriert und entsprechend gezählt, wenn er oberhalb eines definierten Schwellwerts THR, d.h. im Wesentlichen einer Energieschwelle, liegt. Ein Ereignis wird gezählt, indem ein Zählerstand des Zählelement 13 um eine Zähleinheit hochgezählt wird, wenn das erzeugte Signal über dem einstellbaren Schwellwert THR liegt.

Der einstellbare Schwellwert THR ist dabei üblicherweise über einen Komparator 19 einstellbar. Der Schwellwert THR kann prinzipiell auch fest analog vorgegeben sein, wird aber im Allgemeinen über z. B. einen DAC (Digital-Analog-Wandler = digital-to-analog-converter) angelegt. Der Schwellwert THR ist damit üblicherweise zumindest in einem gewissen Energiebereich variabel einstellbar. Der Schwellwert THR kann entweder pixelweise lokal (mittels des Komparators und des DAC), für Gruppen von Pixelelementen oder global im Röntgendetektor 1 für alle Pixelelemente 5 des Röntgendetektors 1 einstellbar sein. In dem Falle, dass zwei, drei oder mehr einstellbare Schwellwerte THR in einem Pixelelement 5 für energieaufgelöste Messungen bereitgestellt sind, wird das erzeugte elektrische Signal entsprechend der unterschiedlichen, vordefinierten Schwellwerte THR in eine oder mehrere Zählelemente 13, welche jeweils mit einer Energieschwelle THR verknüpft sind, eingeordnet, d.h. gezählt.

Zumindest eine Teilzahl der Vielzahl an Pixelelementen 5 des erfindungsgemäßen Röntgendetektors 1 weist erfindungsgemäß zumindest einen mit einen Signaleingang 7, über welchen das elektrische Signal von dem Konverterelement 3 in das Pixelelement 5 eingespeist wird, signaltechnisch gekoppelten konfigurierbaren Zähler 9 auf. Der konfigurierbare Zähler 9 ist ausgebildet entweder ein Pixel-Zählsignal zu zählen, welches auf einem in einem jeweiligen Pixelelement 5 der Teilzahl der Vielzahl an Pixelelementen 5 direkt eingegangenen Signal basiert oder ein Koinzidenz-Zählsignal zu zählen, welches auf dem in dem jeweiligen Pixelelement 5 direkt eingegangenen Signal und auf einem koinzidierend auftretenden Signal mindestens eines weiteren Pixelelements 5 der Vielzahl an Pixelelementen 5 basiert.

Der konfigurierbare Zähler weist einen konfigurierbaren Multiplexer 11 zur Konfiguration des konfigurierbaren Zählers 9 auf und ein damit gekoppeltes Zählelement 13. Der konfigurierbare Multiplexer 11 kann ausgebildet sein auf einen Steuerbefehl einer Steuereinheit 53 hin den konfigurierbaren Zähler 9 entweder in der ersten Einstellung zum Zählen der Pixel-Zählsignale oder in der zweiten Einstellung zum Zählen der Koinzidenz-Zählsignale zu konfigurieren. Der zumindest eine konfigurierbare Zähler 9 kann für jedes Pixelelement 5 der Teilzahl der Vielzahl an Pixelelementen 5 individuell und/oder jeweils für eine Gruppe an Pixelelementen 5 der Teilzahl der Vielzahl an Pixelelementen 5 gemeinsam konfigurierbar sein.

Erfindungsgemäß weist zumindest eine Teilzahl der Vielzahl an Pixelelementen 5 weist zumindest einen konfigurierbaren Zähler 9 auf. Die Pixelelemente 5 der Teilzahl der Vielzahl an Pixelelementen 5 können ebenso auch mehr als einen konfigurierbaren Zähler 9 aufweisen, beispielsweise zwei oder drei. Neben dem zumindest einem konfigurierbaren Zähler 9 kann ein Pixelelement der Teilzahl der Vielzahl an Pixelelementen 5 außerdem auch zumindest einen nicht-konfigurierbaren Zähler aufweisen. Neben derart konfigurierbaren Pixelelementen kann der Röntgendetektor auch nicht-konfigurierbare Pixelelemente aufweisen.

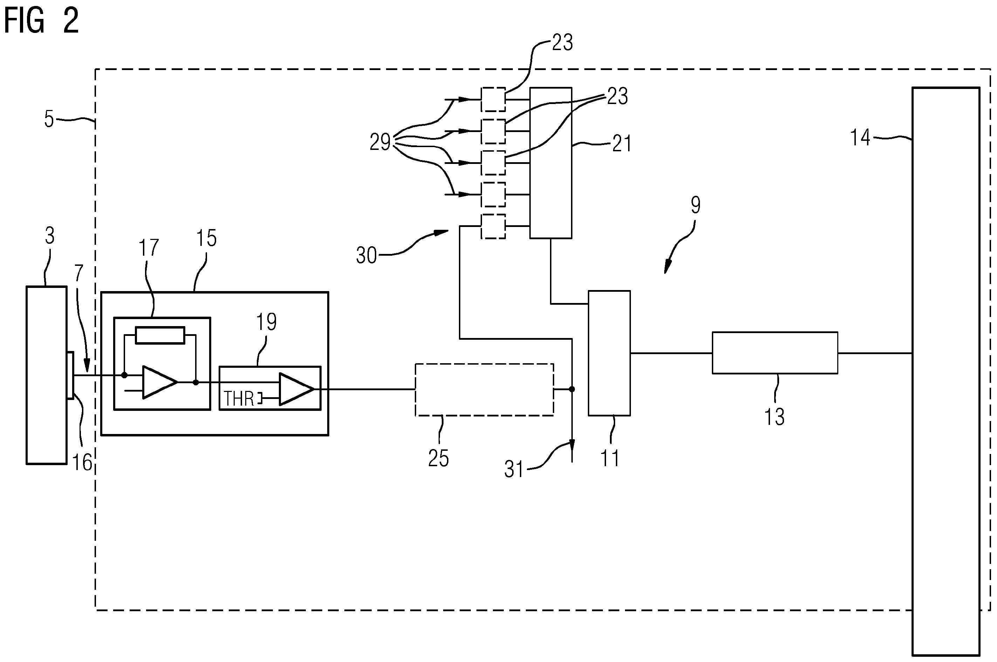

Die Fig. 2 zeigt ein Schema zur Veranschaulichung einer signaltechnischen Verschaltung eines konfigurierbaren Pixelelements 5 der Teilzahl der Vielzahl an Pixelelementen 5 eines photonenzählenden Röntgendetektors 1 gemäß einer ersten Ausführungsvariante mit einem konfigurierbaren Zähler 9. Exemplarisch ist lediglich ein einzelnes Pixelelement 5 der Teilzahl der Vielzahl an Pixelelementen 5 gezeigt. Die Verschaltung kann jedoch leicht auch auf weitere Pixelelemente 5 übertragen werden.

Der Signaleingang 7 des Pixelelements ist über die Sensor-Pixelelektrode 16 mit dem dem Pixelelement 5 zugeordneten sensitiven Detektionsvolumen im Konverterelement 3 signaltechnisch gekoppelt. Das hier dargestellte Pixelelement 5 der Teilzahl der Vielzahl an Pixelelementen 5 weist für eine vorteilhaft praktische Umsetzung weiterhin eine mit dem Signaleingang 7 signaltechnisch gekoppelte Wandlungsvorrichtung 15 auf, umfassend zumindest einen Signalverstärker 17 zur Verstärkung des durch die eintreffende Röntgenstrahlung generierten Signals und umfassend zumindest einen Komparator 19. Mittels des Komparators 19 wird das verstärkte Signal mit einem einstellbaren Schwellwert THR verglichen. Übersteigt das verstärkte Signal den Schwellwert THR des Komparators 19 wird am Signalausgang des Komparators 19 ein Pixel-Zählsignal generiert, welches auf dem über den Signaleingang direkt in einem Pixelelement 5 eingegangen Signal basiert.

Der Signalausgang des Komparators 19 ist weiterhin mit dem konfigurierbaren Zähler 9 signaltechnisch gekoppelt. Der konfigurierbare Zähler 9 umfasst erfindungsgemäß einen konfigurierbaren Multiplexer 11 und ein mit dem Signalausgang des Multiplexers 11 gekoppeltes Zählelement 13. In einer ersten Einstellung des Multiplexers 11 wird das Ausgangssignal des Komparators 19, d.h. das Pixel-Zählsignal, an das Zählelement 13 weitergegeben, welches die Anzahl an eingegangenen Pixel-Zählsignalen zählt und zumindest vorläufig abspeichert.

Weiterhin weist das hier dargestellte Pixelelement 5 der Teilzahl der Vielzahl an Pixelelementen 5 eine Koinzidenzlogik 21 auf. Die hier exemplarisch ausgeführte Koinzidenzlogik 21 ist zum einem über einen Signaleingang 30 ebenfalls mit dem Signalausgang des Komparators 19 des hier dargestellten Pixelelements 5 signaltechnisch verknüpft. Des Weiteren ist die Koinzidenzlogik 21 mit mindestens einen weiteren Pixelelement 5 der Vielzahl an Pixelelementen 5 über einen Signaleingang 29 der Koinzidenzlogik 21 signaltechnisch gekoppelt. In dem hier gezeigten Beispiel weist die Koinzidenzlogik 21 vier weitere Signaleingänge 29 auf. Insbesondere ist in diesem Beispiel jeder der vier Signaleingänge 29 mit einem weiteren Pixelelement 5 der Vielzahl an Pixelelementen 5 signaltechnisch gekoppelt. Die Koinzidenzlogik 21 ist ausgebildet ein Koinzidenz-Zählsignal, welches auf dem in dem Pixelelement 5 der Vielzahl an Pixelelementen 50 direkt eingegangenem Signal und auf einem koinzidierend auftretenden Signal mindestens eines weiteren verknüpften Pixelelements 5 der Vielzahl an Pixelelementen 5 basiert, zu bilden.

Die in diesem Beispiel vier weiteren Pixelelemente 5 der Vielzahl an Pixelelementen 5 können beispielsweise die vier direkt benachbarten Pixelelemente des betrachteten Pixelelements 5 umfassen. Es kann jedoch auch eine andere Anzahl oder Auswahl an Pixelelementen 5 der Vielzahl an Pixelelementen 5 mit der Koinzidenzlogik 21 signaltechnisch gekoppelt sein. Beispielsweise sind neben den direkt benachbarten Pixelelementen 5 außerdem die diagonal benachbarten Pixelelemente 5 des jeweiligen Pixelelements 5 der Teilzahl der Vielzahl an Pixelelementen 5 mit der Koinzidenzlogik gekoppelt. Bevorzugt basiert das Koinzidenz-Zählsignal im Allgemeinen auf dem in dem jeweiligen Pixelelement 5 der Teilzahl der Vielzahl an Pixelelementen 5 direkt eingegangenem Signal und auf koinzidierend auftretenden Signalen von zwischen einem und 24 weiteren Pixelelementen 5 der Vielzahl an Pixelelementen 5. Besonders bevorzugt basiert das Koinzidenz-Zählsignal auf koinzidierend auftretenden Signalen von zwischen 1 und 8 weiteren Pixelelementen 5 der Vielzahl an Pixelelementen 5.

Insbesondere kann die Anzahl und/oder Auswahl an weiteren Pixelelementen 5 der Vielzahl an Pixelelementen 5, auf welchem das Koinzidenz-Zählsignal eines jeweiligen Pixelelements 5 der Teilzahl der Vielzahl an Pixelelementen 5 basiert für unterschiedliche Pixelelemente 5 der Vielzahl an Pixelelementen 5 unterschiedlich sein.

In einer Ausgestaltungsvariante ist insbesondere die Anzahl und/oder Auswahl an weiteren Pixelelementen 5 der Vielzahl an Pixelelementen 5, auf welchem das Koinzidenz-Zählsignal für ein innerhalb der Matrix an Pixelelementen 5 randseitig angeordnetes Pixelelement 5 basiert, verschieden von der Anzahl und/oder Auswahl an weiteren Pixelelementen 5 der Vielzahl an Pixelelementen 5, auf welchem das Koinzidenz-Zählsignal für ein innerhalb der Matrix an Pixelelementen 5 mittig angeordnetes Pixelelement 5 basiert. Randseitig angeordnete Pixelelemente weisen beispielweise weniger benachbarte Pixelelemente auf als mittig angeordnete Pixelelemente. Außerdem können unterschiedliche Randbedingungen hinsichtlich beispielsweise der Ausbildung des elektrischen Feldes im Konverterelement vorliegen, welche berücksichtigt werden können.

Die Anzahl und/oder Auswahl an weiteren Pixelelementen 5 der Vielzahl an Pixelelementen 5 kann in Ausführungsvarianten für eine besonders flexible und anpassungsfähige Umsetzung des Röntgendetektors konfigurierbar gestaltet sein. Beispielsweise kann die Anzahl und Auswahl mittels einer Steuereinheit ansteuerbar und damit anpassbar gestaltet sein. Sie kann jedoch auch nach Bereitstellung des photonenzählenden Röntgendetektors 1 fest vorgegeben sein.

Die Koinzidenzlogik 21 ist gemäß einer vorteilhaft zweckmäßigen Ausgestaltung insbesondere jeweils mit zumindest einem Komparator 19 des mindestens einen weiteren Pixelelements 5 der Vielzahl an Pixelelementen 5 signaltechnisch gekoppelt. Das Koinzidenz-Zählsignal basiert dann auf den Ausgangssignalen der mit der Koinzidenzlogik 21 signaltechnisch gekoppelten Komparatoren 19 des betrachteten Pixelelements 5 und des zumindest einen weiteren verknüpften Pixelelements 5, in diesem konkreten Fall der vier weiteren Pixelelemente 5.

Die jeweils mit einer Koinzidenzlogik 21 gekoppelten Komparatoren 19 können auf einen Schwellwert eingestellt sein, welcher jeweils die gleiche Energieschwelle repräsentiert. Beispielsweise weist der Komparator 19 des hier dargestellten Pixelelements 5 ebenso wie die mit dem Koinzidenzlogik 21 gekoppelten weiteren Komparatoren der vier weiteren Pixelelemente 5 jeweils die gleiche Energieschwelle, beispielsweise 40keV oder 60keV, auf. Daneben kann es natürlich auch andere signaltechnische Verschaltungen geben, wobei beispielsweise unterschiedlich eingestellte Energieschwellen verknüpft werden.

Der Signalausgang der Koinzidenzlogik 21 ist ebenfalls mit dem konfigurierbaren Multiplexer 11 signaltechnisch gekoppelt. In einer zweiten Einstellung des konfigurierbaren Multiplexers 11 wird anstelle des Ausgangssignal des Komparators 19 das Ausgangssignal der Koinzidenzlogik 21, d.h. das Koinzidenz-Zählsignal, an das Zählelement 13 ausgegeben, gezählt und zumindest vorläufig abgespeichert.

Anschließend kann das Zählelement 13 über eine Ansteuer- und Ausleseeinheit 14 ausgelesen werden. Die Ansteuer- und Ausleseeinheit 14 kann beispielsweise auch in einer peripheren Elektronik 65 implementiert sein.

Außerdem ist ein mit dem Komparator 19 des gezeigten Pixelelements 5 verknüpfter, weiterer Signalausgang 31 vorgesehen, welche wiederum für die Bereitstellung eines Eingangssignals einer Koinzidenzlogik 21 eines zweiten hier nicht dargestellten Pixelelements 5 der Teilzahl der Vielzahl an Pixelelementen 5 dienen kann.

Weiterhin kann das hier dargestellte Pixelelement 5 der Teilzahl der Vielzahl an Pixelelementen 5 zumindest ein Einstellelement 23 zur Laufzeitanpassung, Pulslängenanpassung oder zur Verzögerung eines Eingangssignals in die zumindest eine Koinzidenzlogik 21 aufweisen. In diesem Beispiel ist für jeden Signaleingang 29 der Koinzidenzlogik 21 ein Einstellelement 23 zur Laufzeitanpassung oder zur Verzögerung der Eingangssignale angedeutet. Die Einstellelemente 23 können jeweils konfigurierbar, d.h. einstellbar bzw. ansteuerbar mittels einer Steuereinheit 53 sein, so dass diese auch nach Bereitstellung des photonenzählenden Röntgendetektors 1 gegebenenfalls angepasst werden können. Die Einstellelemente 23 können dazu dienen Signalunterschiede, beispielweise unterschiedliche Laufzeiten durch unterschiedliche lange Signalleitungen auszugleichen, oder auch um ein Zeitfenster festzulegen, innerhalb welchem auftretende Signale als koinzident gelten, d.h. dem gleichen Photonenereignis zugeordnet werden.

In Ausführungsvarianten kann das Pixelelement 5 der Teilzahl der Vielzahl an Pixelelementen 5 außerdem zumindest ein elektronisches Element 25 zur Verhinderung einer Paralyse des zumindest einen konfigurierbaren Zählers 9 umfasst. Das elektronisches Element 25 kann, wie hier in der schematischen Darstellung angedeutet, einem jeweiligen konfigurierbaren Multiplexer 11 signaltechnisch vorgeschaltet sein. Das heißt, es kann jeweils zwischen dem Signalausgang des mit dem konfigurierbaren Zähler 9 gekoppelten Komparators 19 und dem Signaleingang des konfigurierbaren Multiplexer 11 angeordnet sein. Ebenso kann das zumindest eine elektronisches Element 25 zur Verhinderung einer Paralyse jeweils dem konfigurierbaren Multiplexer 11 signaltechnisch nachgeschaltet sein. Das heißt, es kann zwischen dem Signalausgang des jeweiligen konfigurierbaren Multiplexers 11 und dem Signaleingang des jeweiligen Zählelements 13 des konfigurierbaren Zählers 9 angeordnet sein. Vorteilhaft kann ein verbessertes Hochflussverhalten des Pixelelements 5 erreicht werden.

Das elektronische Element 25 kann beispielsweise als Pile-Up Trigger (siehe beispielsweise Kraft et al. "Experimental evaluation of the pile-up trigger method in a revised quantum-counting CT detector", Proc. SPIE 8313, Medical Imaging 2012: Physics of Medical Imaging, 83134A (2012); https://doi.org/10.1117/12.911231) oder als sogenannter "instant retrigger" (Loeliger et al. "The new PILATUS3 ASIC with instant retrigger capability", 2012 IEEE Nuclear Science Symposium and Medical Imaging Conference Record (NSS/MIC) (2012); https://doi.org/610-615.10.1109/NSSMIC.2012.6551180) ausgebildet sein.

Ein Pixelelement 5 der Teilzahl der Vielzahl an Pixelelementen 5 kann darüber hinaus noch weitere Elemente zur Verarbeitung der erzeugten Signale aufweisen, welche hier nicht weiter dargestellt sind. Insbesondere kann ein Pixelelement 5 der Teilzahl der Vielzahl an Pixelelementen 5 eine Mehrzahl an Komparatoren 19 mit jeweils einem einstellbaren Schwellwert THR aufweisen, welche jeweils mit dem Signalverstärker 17 signaltechnisch gekoppelt sind. Derart können mehrere Energieschwellen bereitgestellt werden. Insbesondere können die weiteren Komparatoren 19 entweder mit weiteren konfigurierbaren Zählern 9 oder auch mit nicht-konfigurierbaren, regulären Zählern umfassend ein Zählelement 13 gekoppelt sein.

Beispielweise kann jeder konfigurierbare Zähler per Konfigurationsbit von einem Zählmodus auf den anderen Zählmodus umgewidmet werden.

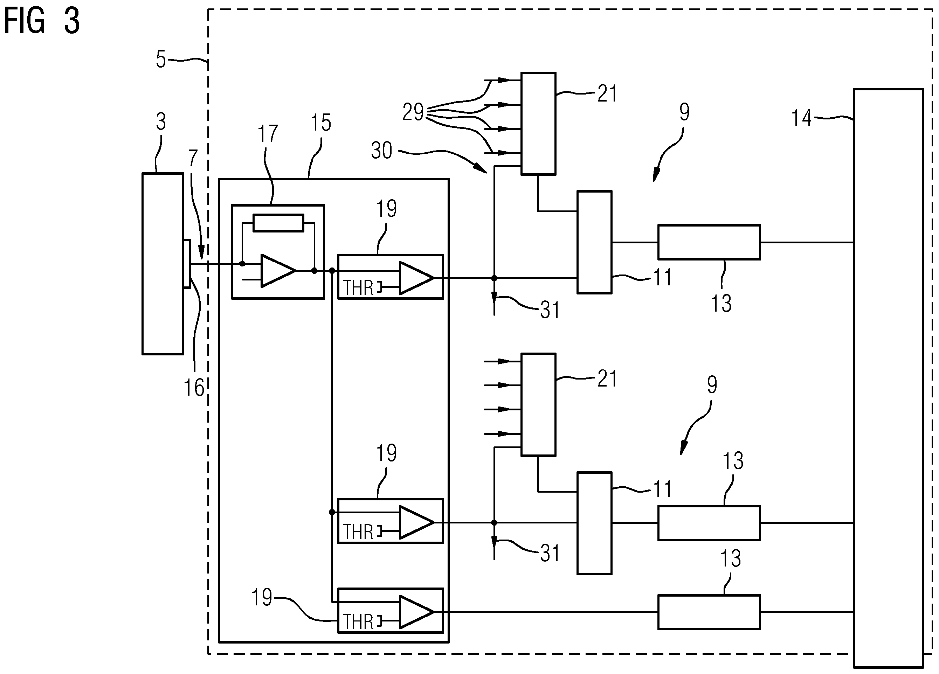

Die Fig. 3 zeigt ein Schema zur Veranschaulichung einer signaltechnischen Verschaltung eines konfigurierbaren Pixelelements 5 der Teilzahl der Vielzahl an Pixelelementen 5 eines photonenzählenden Röntgendetektors 1 gemäß einer zweiten Ausführungsvariante mit einem konfigurierbaren Zähler 9.

Hier weist das Pixelelement 5 der Teilzahl der Vielzahl an Pixelelementen 5 mehrere Komparatoren 19 mit jeweils einem einstellbaren Schwellwert THR auf. Dabei sind zwei der Komparatoren 19 jeweils mit einem konfigurierbaren Zähler 9 und jeweils einer Koinzidenzlogik 21 signaltechnisch verbunden. Ein dritter Komparator 19 ist dagegen lediglich mit einem regulären Zählelement 13 signaltechnisch gekoppelt. Der dritte Komparator 19 ist damit lediglich ausgebildet Pixel-Zählsignale basierend auf dem Ausgangssignal des gekoppelten Komparators 19 und in Abhängigkeit dessen Schwellwert THR zu zählen. Die beiden anderen Komparatoren können je nach Einstellung des jeweiligen konfigurierbaren Zählers 9 entweder gemäß der ersten Einstellung Pixel-Zählsignale basierend auf dem Signalausgang ihres zugeordneten Komparators 19 zählen oder gemäß der zweiten Einstellung Koinzidenz-Zählsignale basierend auf der Ausgangssignalen der jeweilig gekoppelten Koinzidenzlogik 21 zählen. In anderen Ausführungsvarianten können auch alle Komparatoren 19 oder nur ein Komparator 19 mit einem konfigurierbaren Zähler 9 verbunden sein.

Bevorzugt ist zumindest ein Komparator 19 mit einem konfigurierbaren Zähler 9 gekoppelt. Sind die restlichen Komparatoren dann mit regulären Zählern ausgestattet sind umfasst diese Implementation einen vorteilhaft geringen Routingaufwand und einen geringen Leistungsverbrauch. Vorteilhaft ist zumindest derjenige Komparator 19 mit einem konfigurierbaren Zähler 9 gekoppelt welcher auf den im Vergleich zu den anderen Komparatoren 19 des Pixelelements niederenergetischsten Schwellwert THR eingestellt ist bzw. auf den niederenergetischsten Schwellwert THR einstellbar ist. Koinzidenzinformation in Abhängigkeit der niederenergetischsten eingestellten Energieschwelle ermöglicht mit einem möglichst geringen Aufwand bereits weitreichende Korrekturmöglichkeiten. Vorteilhaft kann dies kombiniert mit der Möglichkeit sein, Schwellwerte THR von zumindest einem Teil der Komparatoren 19 eines Pixelelement 5 zumindest in überlappenden Energiebereichen einzustellen. Derart kann ermöglicht werden, dass die Schwellwerte zweier Komparatoren 19 eine im Wesentlichen gleiche Energieschwelle repräsentieren. Derart kann in einem Pixelelement 5 Koinzidenzinformation in Abhängigkeit einer Energieschwelle gesammelt werden, ohne auf Pixel-Zählsignale in Abhängigkeit derselben Energieschwelle verzichten zu müssen.

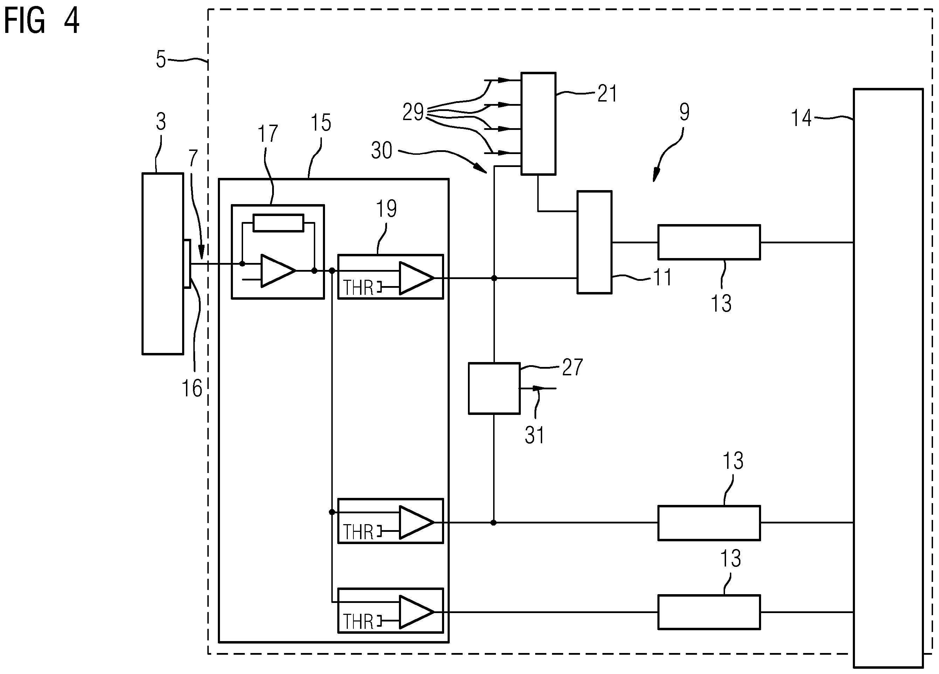

Die Fig. 4 zeigt ein Schema zur Veranschaulichung einer signaltechnischen Verschaltung eines konfigurierbaren Pixelelements 5 der Teilzahl der Vielzahl an Pixelelementen 5 eines photonenzählenden Röntgendetektors 1 gemäß einer dritten Ausführungsvariante mit einem konfigurierbaren Zähler 9.

Dabei weist das hier dargestellte Pixelelement 5 der Teilzahl der Vielzahl an Pixelelementen 5 ein weiteres Schaltelement 27 auf. Das weitere Schaltelement ist mit den Signalausgängen zwei der Komparatoren 19 des betrachteten Pixelelements 5 signaltechnisch verknüpft. Das Schaltelement 27, beispielsweise ebenfalls umfassend einen Multiplexer, ist dabei ausgebildet entweder das Ausgangssignal des ersten Komparators 19 oder das Ausgangssignal des zweiten Komparators 19 über den Signalausgang 31 an zumindest ein zweites, nicht dargestelltes Pixelelement 5 der Teilzahl der Vielzahl an Pixelelementen 5 auszugeben. Das ausgegebene Ausgangssignal kann dann entsprechend als Eingangssignal über einen Signaleingang 29 in eine Koinzidenzlogik 21 eines hier nicht dargestellten Pixelelements 5 der Teilzahl der Vielzahl an Pixelelementen 5 eingespeist werden.

Insbesondere ist damit die zumindest eine Koinzidenzlogik 21 eines jeweiligen Pixelelements 5 der Teilzahl der Vielzahl an Pixelelementen 5 ist mit mehr als einem Komparator 19 des mindestens einen weiteren Pixelelements 5 der Vielzahl an Pixelelementen 5 signaltechnisch gekoppelt.

Dabei ist mittels des Schaltelements 27 insbesondere konfigurierbar bzw. umschaltbar, welcher Komparator 19 als Eingangssignal der Koinzidenzlogik 21 dient. Damit ist insbesondere einstellbar und leicht umschaltbar, basierend auf welchem Komparator 19 ein Koinzidenz-Zählsignal basiert. Vorteilhaft kann eine weitere Flexibilisierung bei gleichzeitiger Ressourcenschonung erreicht werden.

Insbesondere in dem Falle, wenn der Schwellwert THR der Komparatoren 19 eines Pixelelements jeweils nur auf einen eingeschränkten Energiebereich und möglicherweise nicht überlappend mit den Energiebereichen weiterer Komparatoren einstellbar ist, kann hiermit eine erhöhte Flexibilität für die Sammlung von Koinzidenzinformation in Abhängigkeit unterschiedlicher Energieschwellen erreicht werden.

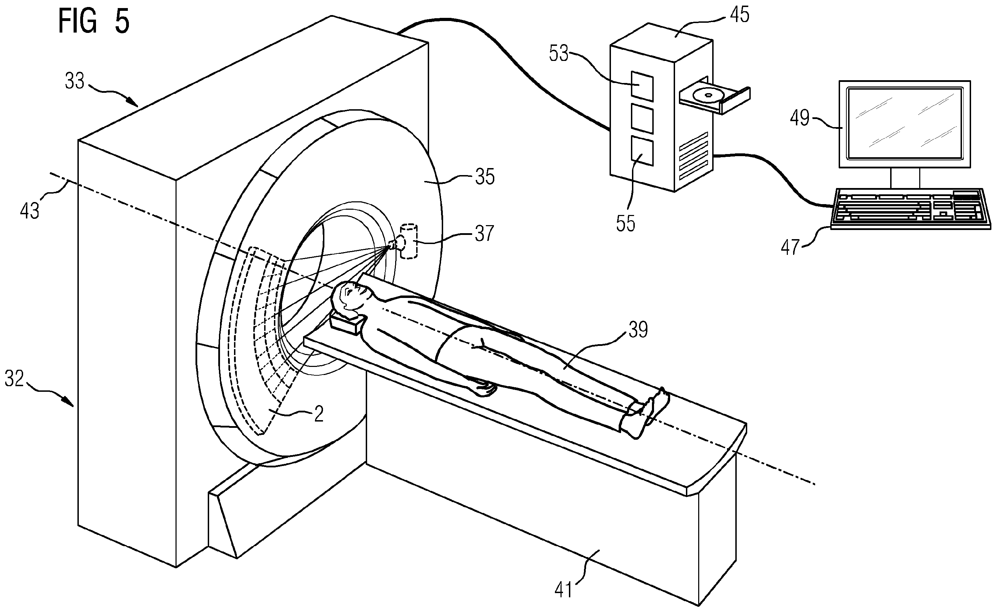

Die Fig. 5 zeigt eine beispielhafte Ausführungsform eines erfindungsgemäßen medizinischen Bildgebungsgeräts in Form eines Computertomographie-Systems 32. Das Computertomographie-System 32 weist eine Gantry 33 mit einem Rotor 35 auf. Der Rotor 35 umfasst eine Strahlungsquelle oder Röntgenquelle 37 und eine Detektorvorrichtung 2. Die Detektorvorrichtung 2 weist mindestens einen erfindungsgemäßen Röntgendetektor 1 auf. Die Detektorvorrichtung 2 kann ein Detektormodul 51 mit einer Anzahl an Röntgendetektoren 1 aufweisen. Das Objekt 39, hier der Patient, ist auf der Patientenliege 41 gelagert und ist entlang der Rotationsachse z 43 durch die Gantry 33 bewegbar. Im Allgemeinen kann das Objekt 39 beispielsweise einen tierischen Patienten und/oder einen menschlichen Patienten umfassen. Zur Steuerung des medizinischen Bildgebungsgeräts und/oder zur Erstellung eines Röntgenbilddatensatzes basierend auf den gezählten Pixel-Zählsignalen und/oder Koinzidenz-Zählsignalen ist eine Systemsteuerung in Form einer Recheneinheit 45 vorgesehen.

Die Recheneinheit 45 kann eine Steuereinheit 53 zur Steuerung des zumindest einen Röntgendetektors 1 umfassen. Insbesondere kann mittels der Steuereinheit 53 der zumindest eine konfigurierbare Zähler 9 der Pixelelemente 5 der Teilzahl der Vielzahl an Pixelelementen 5 angesteuert und damit konfiguriert werden.

Die Steuereinheit 53 und/oder die Recheneinheit 45 kann in Form eines Computers, eines Mikrocontrollers oder eines integrierten Schaltkreises umgesetzt sein. Die Steuereinheit 53 und/oder die Recheneinheit 45 kann Hardware-Elemente oder Software-Elemente aufweisen, beispielsweise einen Mikroprozessor oder ein sogenanntes FPGA (englisches Akronym für "Field Programmable Gate Array"). Es kann sich auch um einen realen oder virtuellen Verbund von Computern handeln (ein englischer Fachbegriff für einen realen Verbund ist "Cluster", ein englischer Fachbegriff für einen virtuellen Verbund ist "Cloud").

Basiert das Konfigurieren des zumindest einen konfigurierbaren Zählers 9 der Pixelelemente 5 der Teilzahl der Vielzahl an Pixelelementen 5 auf einem oder mehreren Parametern, kann beispielsweise der oder die Parameter z. B. aus einem Speicher 55 oder der Systemsteuerung des medizinischen Bildgebungsgerät abgefragt oder aber direkt ermittelt oder gemessen werden. Die Abfrage und/oder Ermittlung kann insbesondere automatisch mittels der Steuereinheit 53 ausgeführt werden.

Im Falle eines computertomographie-Systems wird üblicherweise aus einer Vielzahl an Winkelrichtungen ein (Roh-) Röntgenbilddatensatz des Objekts mittels des Röntgendetektors aufgenommen. Anschließend kann basierend auf dem (Roh-) Röntgenbilddatensatz mittels eines mathematischen Verfahrens, beispielsweise umfassend eine gefilterte Rückprojektion oder ein iteratives Rekonstruktionsverfahren, ein finaler Röntgenbilddatensatz rekonstruiert werden.

Der (Roh-)Röntgenbilddatensatz kann dabei je nach signaltechnischer Verschaltung der Pixelelemente 5 der Vielzahl an Pixelelementen 5 und nach jeweiliger Konfiguration der konfigurierbaren Zähler der Pixelelemente 5 der Teilzahl der Vielzahl an Pixelelementen 5 (Roh-)Röntgenbilder basierend auf gezählten Anzahlen an Pixel-Zählsignale, gezählten Anzahlen an Koinzidenz-Zählsignale oder basierend auf beidem umfassen. Die Anzahlen an Pixel-Zählsignalen und die Anzahlen an Koinzidenz-Zählsignale bzw. die darauf basierenden (Roh-)Röntgenbilder können außerdem in Abhängigkeit mehrerer Schwellwerte, d.h. Energieschwellen, vorliegen. Insbesondere können gezählte Anzahlen an Koinzidenz-Zählsignalen für eine Korrektur der gezählten Anzahlen an Pixel-Zählsignalen dienen, wobei ein daraus resultierender Röntgenbilddatensatz dann auf den korrigierten Anzahlen basieren kann. Eine Korrektur kann auch erste auf Bildebene durchgeführt werden.

Des Weiteren ist eine Eingabeeinrichtung 47 und eine Ausgabeeinrichtung 49 mit der Rechnereinheit 45 verbunden. Die Eingabeeinrichtung und die Ausgabeeinrichtung können beispielsweise eine Interaktion, beispielsweise eine manuelle Konfiguration, eine Bestätigung oder ein Auslösen eines Verfahrensschritts durch einen Anwender ermöglichen.

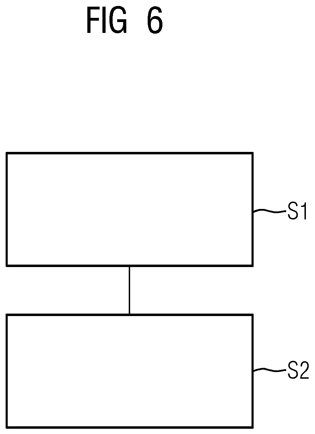

Fig. 6 zeigt einen schematischen Verfahrensablauf eines Verfahrens zum Betreiben eines erfindungsgemäßen photonenzählenden Röntgendetektors zur Aufnahme eines Röntgenbilddatensatzes eines von einer Röntgenstrahlung durchstrahlten Objektes 39, wobei in einem ersten Zählmodus der zumindest eine konfigurierbare Zähler 9 ein Pixel-Zählsignal zählt, welches auf einem in jedem Pixelelement 5 der Teilzahl der Vielzahl an Pixelelementen 5 direkt eingegangenen Signal basiert und in einem zweiten Zählmodus der konfigurierbare Zähler 9 ein Koinzidenz-Zählsignal zählt, welches auf dem in dem jeweiligen Pixelelement 5 direkt eingegangenen Signal und auf einem koinzidierend auftretenden Signal mindestens eines weiteren Pixelelements 5 der Vielzahl an Pixelelementen 5 basiert, und wobei zwischen dem ersten und dem zweiten Zählmodus umgeschaltet werden kann.

Schritt S1 in Fig. 6 umfasst ein Bereitstellen des photonenzählenden Röntgendetektors 1 in einer ersten Konfiguration der Pixelelemente 5 der Teilzahl der Vielzahl an Pixelelementen 5, wobei der zumindest eine konfigurierbare Zähler 9 der Pixelelemente 5 der Teilzahl der Vielzahl an Pixelelementen 5 entweder für den ersten oder für den zweiten Zählmodus konfiguriert ist.

Schritt S2 in Fig. 6 umfasst ein Konfigurieren des photonenzählenden Röntgendetektors 1 aus Schritt 1, wobei zumindest ein Teil der Pixelelemente 5 der Teilzahl der Vielzahl an Pixelelementen 5 der Zählmodus umgeschaltet wird, indem der zumindest eine konfigurierbare Zähler 9 des zumindest eines Teils der Pixelelemente 5 der Teilzahl der Vielzahl an Pixelelementen 5 von der ersten in die zweite Einstellung umgeschaltet wird.

Dabei kann vorgesehen sein, dass individuell für jedes Pixelelement der Teilzahl der Vielzahl an Pixelelementen 5 der zumindest eine konfigurierbare Zähler konfiguriert wird. Ebenso kann eine Konfiguration in Gruppen von Pixelelementen 5 der Teilzahl der Vielzahl an Pixelelementen vorgesehen sein. Eine Gruppe kann dabei auch die gesamte zumindest Teilzahl und damit auch die gesamte Vielzahl an Pixelelementen umfassen.

Das Konfigurieren kann auch umfassen, dass eine Auswahl oder Anzahl an weiteren Pixelelementen individuell für jedes Pixelelement der Teilzahl der Vielzahl an Pixelelementen 5 oder gruppenbasiert konfiguriert wird. Das Konfigurieren kann auch umfassen, dass konfiguriert wird, auf welchen Komparatoren und damit Schwellwerten das Koinzidenz-Zählsignal, welches mittels des konfigurierbaren Zählers gezählt wird, basiert.

Es kann außerdem ein Schritt der Abfrage und/oder Ermittlung von zumindest einem Parameter des medizinischen Bildgebungsgeräts, des Röntgendetektors und/oder eines jeweiligen Pixelelements vorgesehen sein.

Es kann vorgesehen sein, dass die Konfiguration der Pixelelemente 5 der Teilzahl der Vielzahl an Pixelelementen 5 von einem oder mehreren Parametern abhängig ist. Der oder die Parameter können z. B. aus einem Speicher oder einer Systemsteuerung des Röntgensystems abgefragt oder aber direkt ermittelt, beispielsweise mittels des Röntgendetektors gemessen, werden. Der zumindest eine Parameter kann beispielsweise auf einem Röntgenfluss basieren. Es kann auch eine Steuerung z. B. abhängig von der für die Applikation zu erwartenden Zählrate (Dosis/Zeiteinheit) vorgesehen sein. Der Parameter kann auch eine Position eines Pixelelements umfassen. Der Parameter kann auch anderweitig bestimmt sein.

Der Schritt des Konfigurierens S2 kann insbesondere automatisch mittels einer Steuereinheit 53 durchgeführt werden, indem der konfigurierbare Zähler automatisch angesteuert und konfiguriert wird. Bei Bedarf kann auch eine manuelle Aktivierung vorgesehen sein. Die automatische Ansteuerung kann in Verbindung mit dem oder den ermittelten Parametern erfolgen. Auch kann eine automatische Durchführung eines Schritts der Abfrage und/oder Ermittlung mittels einer Steuereinheit 53 vorgesehen sein.

Weiterhin kann das Verfahren eine pixelweise Umwandlung der das Objekt durchstrahlenden und auf dem Röntgendetektor auftreffenden Röntgenstrahlung in elektrische Signale in den Pixelelementen umfassen. Weiterhin kann das Verfahren eine Umwandlung der elektrischen Signale in Pixel-Zählsignale und/oder in Koinzidenz-Zählsignale, je nach Konfiguration und Verschaltung, und eine Speicherung der gezählten Anzahlen in den Pixelelementen umfassen. Anschließend kann ein Auslesen der gezählten Anzahlen an Pixel-Zählsignalen und/oder Koinzidenz-Zählsignalen und ein Erstellen eines oder mehrere das Objekt repräsentierender Bilddatensätze vorgesehen sein.

Nach einer Ausgestaltungsvariante des Verfahrens können für den Fall, dass sowohl Pixel-Zählsignale als auch Koinzidenz-Zählsignale gezählt werden, die Anzahlen an Koinzidenz-Zählsignalen bzw. Röntgenbilder, welche auf den Anzahlen an Koinzidenz-Zählsignalen basieren, zur Korrektur der Anzahlen an Pixel-Zählsignalen bzw. Röntgenbilder, welche auf den Anzahlen an Pixel-Zählsignalen basieren verwendet werden.

## Patentansprüche

1. Photonenzählender Röntgendetektor (1) zur Aufnahme eines Röntgenbilddatensatzes eines von einer Röntgenstrahlung durchstrahlten Objektes (39), aufweisend ein Konverterelement (3) ausgebildet eintreffende Röntgenstrahlung in ein elektrisches Signal umzuwandeln und eine Matrix mit einer Vielzahl an Pixelelementen (5), wobei
- zumindest eine Teilzahl der Vielzahl an Pixelelementen einen Signaleingang (7) und zumindest einen damit signaltechnisch gekoppelten konfigurierbaren Zähler (9) aufweist, und
- der konfigurierbare Zähler (9) ausgebildet ist wahlweise entweder ein Pixel-Zählsignal zu zählen, welches auf einem in jedem Pixelelement (5) der Teilzahl der Vielzahl an Pixelelementen direkt eingegangenen Signal basiert oder ein Koinzidenz-Zählsignal zu zählen, welches auf dem in dem jeweiligen Pixelelement (9) direkt eingegangenen Signal und auf einem koinzidierend auftretenden Signal mindestens eines weiteren Pixelelements (5) der Vielzahl an Pixelelementen basiert,
**dadurch gekennzeichnet, dass** der zumindest eine konfigurierbare Zähler (9) einen einem Zählelement (13) vorgeschalteten konfigurierbaren Multiplexer (11) aufweist, welcher zumindest eine erste und eine zweite Einstellung aufweist, wobei in der ersten Einstellung des Multiplexers (11) das Pixel-Zählsignal mittels des Zählelements (13) gezählt und gespeichert wird und wobei in der zweiten Einstellung des Multiplexers (11) das Koinzidenz-Zählsignal mittels des Zählelements (13) gezählt und gespeichert wird.

2. Photonenzählender Röntgendetektor (1) nach Anspruch 1, wobei der zumindest eine konfigurierbare Zähler (9) für jedes Pixelelement (5) der Teilzahl der Vielzahl an Pixelelementen (5) individuell oder jeweils für eine Gruppe an Pixelelementen (5) der Teilzahl der Vielzahl an Pixelelementen (5) gemeinsam konfigurierbar ist.

3. Photonenzählender Röntgendetektor (1) nach einem der Ansprüche 1 oder 2, wobei jedes Pixelelement (5) der Vielzahl an Pixelelementen eine mit dem Signaleingang verbundene Wandlungsvorrichtung (15) mit zumindest einem Signalverstärker (17) und eine Anzahl an Komparatoren (19) mit jeweils einem einstellbaren Schwellwert (THR) aufweist, und wobei bei jedem Pixelelement (5) der Teilzahl der Vielzahl an Pixelelementen zumindest ein Komparator (19) der Anzahl an Komparatoren (19) mit dem zumindest einen konfigurierbaren Zähler (9) signaltechnisch gekoppelt ist.

4. Photonenzählender Röntgendetektor (1) nach Anspruch 3, wobei jedes Pixelelement (5) der Teilzahl der Vielzahl an Pixelelementen außerdem zumindest eine Koinzidenzlogik (21) aufweist, welche mit zumindest einem Komparator (19) des jeweiligen Pixelelements (5) der Teilzahl der Vielzahl an Pixelelementen und mit zumindest einem Komparator (19) des mindestens einen weiteren Pixelelements (5) der Vielzahl an Pixelelementen signaltechnisch gekoppelt ist, wobei das Koinzidenz-Zählsignal auf einem Ausgangssignal der Koinzidenzlogik (21) basiert.

5. Photonenzählender Röntgendetektor (1) nach Anspruch 4, wobei jedes Pixelelement (5) der Vielzahl an Pixelelementen eine Mehrzahl an Komparatoren (19) aufweist, und wobei die zumindest eine Koinzidenzlogik (21) eines Pixelelements (5) der Teilzahl der Vielzahl an Pixelelementen mit mehr als einem Komparator (19) des mindestens einen weiteren Pixelelements (5) der Vielzahl an Pixelelementen signaltechnisch gekoppelt ist.

6. Photonenzählender Röntgendetektor (1) nach Anspruch 4 oder 5, wobei jedes Pixelelement (5) der Teilzahl der Vielzahl an Pixelelementen zumindest ein Einstellelement (23) zur Laufzeitanpassung oder zur Verzögerung eines Eingangssignals in die zumindest eine Koinzidenzlogik (21) aufweist.

7. Photonenzählender Röntgendetektor (1) nach einem der Ansprüche 3 bis 6, wobei jedes Pixelelement (5) der Teilzahl der Vielzahl an Pixelelementen (5) mehrere konfigurierbare Zähler (9) aufweist, welche jeweils mit zumindest einem Komparator (19) des jeweiligen Pixelelements (5) der Teilzahl der Vielzahl an Pixelelementen und mit zumindest einem Komparator (19) des mindestens einen weiteren Pixelelements (5) der Vielzahl an Pixelelementen signaltechnisch gekoppelt sind.

8. Photonenzählender Röntgendetektor (1) nach einem der Ansprüche 1 bis 7, wobei das Koinzidenz-Zählsignal auf dem in dem jeweiligen Pixelelement (5) der Teilzahl der Vielzahl an Pixelelementen direkt eingegangenem Signal und auf koinzidierend auftretenden Signalen von zwischen einem weiteren Pixelelement (5) und 24 weiteren Pixelelementen (5) der Vielzahl an Pixelelementen basiert.

9. Photonenzählender Röntgendetektor (1) nach einem der Ansprüche 1 bis 8, wobei die Anzahl und/oder Auswahl an weiteren Pixelelementen (5) der Vielzahl an Pixelelementen, auf welchem das Koinzidenz-Zählsignal basiert für unterschiedliche Pixelelemente (5) unterschiedlich ist.

10. Photonenzählender Röntgendetektor (1) nach einem der Ansprüche 1 bis 9, wobei die Anzahl und/oder Auswahl an weiteren Pixelelementen (5) der Vielzahl an Pixelelementen, auf welchem das Koinzidenz-Zählsignal für ein innerhalb der Matrix an Pixelelementen (5) randseitig angeordnetes Pixelelement (5) basiert, verschieden ist von der Anzahl und/oder Auswahl an weiteren Pixelelementen (5) der Vielzahl an Pixelelementen, auf welchem das Koinzidenz-Zählsignal für ein innerhalb der Matrix an Pixelelementen (5) mittig angeordnetes Pixelelement basiert.

11. Photonenzählender Röntgendetektor (1) nach einem der Ansprüche 1 bis 10, wobei jedes Pixelelement (5) der Teilzahl der Vielzahl an Pixelelementen außerdem zumindest ein elektronisches Element (25) zur Verhinderung einer Paralyse des zumindest einen konfigurierbaren Zählers (9) umfasst.

12. Medizinisches Bildgebungsgerät aufweisend ein photonenzählenden Röntgendetektor (1) nach einem der vorstehenden Ansprüche.

13. Medizinisches Bildgebungsgerät nach Anspruch 12, wobei das medizinische Bildgebungsgerät als Computertomographie-System ausgebildet ist.

14. Verfahren zum Betreiben eines photonenzählenden Röntgendetektors (1), welcher gemäß einem der Ansprüche 1 bis 11 ausgeführt ist, zur Aufnahme eines Röntgenbilddatensatzes eines von einer Röntgenstrahlung durchstrahlten Objektes (39), wobei in einem ersten Zählmodus der zumindest eine konfigurierbare Zähler (9) eines Pixelelements (5) der Teilzahl der Vielzahl an Pixelelementen (5) ein Pixel-Zählsignal zählt, welches auf einem in dem Pixelelement (5) der Teilzahl der Vielzahl an Pixelelementen (5) direkt eingegangenen Signal basiert und in einem zweiten Zählmodus der konfigurierbare Zähler (9) des Pixelelements (5) ein Koinzidenz-Zählsignal zählt, welches auf dem in dem jeweiligen Pixelelement (5) direkt eingegangenen Signal und auf einem koinzidierend auftretenden Signal mindestens eines weiteren Pixelelements (5) der Vielzahl an Pixelelementen basiert, und wobei wahlweise zwischen dem ersten und dem zweiten Zählmodus umgeschaltet werden kann.

## Claims

1. Photon-counting X-ray detector (1) for acquiring an X-ray image data set of an object (39) penetrated by X-ray radiation, having a converter element (3) designed to convert incident X-ray radiation into an electrical signal, and a matrix with a large number of pixel elements (5), wherein
- at least a partial number of the large number of pixel elements has a signal input (7) and at least one configurable counter (9) coupled thereto for signalling, and
- the configurable counter (9) is designed to optionally count either a pixel count signal, which is based on a signal that has been received directly in each pixel element (5) of the partial number of the large number of pixel elements or a coincidence count signal, which is based on the signal that has been received directly in the respective pixel element (9) and on a coincident signal of at least one further pixel element (5) of the large number of pixel elements,
**characterised in that** the at least one configurable counter (9) has a configurable multiplexer (11) connected upstream of a counting element (13), which has at least one first and one second setting, wherein in the first setting of the multiplexer (11) the pixel count signal is counted and stored by means of the counting element (13) and wherein in the second setting of the multiplexer (11) the coincidence count signal is counted and stored by means of the counting element (13).

2. Photon-counting X-ray detector (1) according to claim 1, wherein the at least one configurable counter (9) can be configured individually for each pixel element (5) of the partial number of the large number of pixel elements (5) or in each case jointly for a group of pixel elements (5) of the partial number of the large number of pixel elements (5).

3. Photon-counting X-ray detector (1) according to one of claims 1 or 2, wherein each pixel element (5) of the large number of pixel elements has a conversion apparatus (15) connected to the signal input, with at least one signal amplifier (17) and a number of comparators (19) each with a settable threshold value (THR), and wherein for each pixel element (5) of the partial number of the large number of pixel elements, at least one comparator (19) of the number of comparators (19) is coupled for signalling to the at least one configurable counter (9).

4. Photon-counting X-ray detector (1) according to claim 3, wherein each pixel element (5) of the partial number of the large number of pixel elements also has at least one coincidence logic (21), which is coupled for signalling to at least one comparator (19) of the respective pixel element (5) of the partial number of the large number of pixel elements and to at least one comparator (19) of the at least one further pixel element (5) of the large number of pixel elements, wherein the coincidence count signal is based on an output signal of the coincidence logic (21).

5. Photon-counting X-ray detector (1) according to claim 4, wherein each pixel element (5) of the large number of pixel elements has a plurality of comparators (19), and wherein the at least one coincidence logic (21) of a pixel element (5) of the partial number of the large number of pixel elements is coupled for signalling to more than one comparator (19) of the at least one further pixel element (5) of the large number of pixel elements.

6. Photon-counting X-ray detector (1) according to claim 4 or 5, wherein each pixel element (5) of the partial number of the large number of pixel elements has at least one setting element (23) for runtime adjustment or for delaying an input signal into the at least one coincidence logic (21).

7. Photon-counting X-ray detector (1) according to one of claims 3 to 6, wherein each pixel element (5) of the partial number of the large number of pixel elements (5) has a plurality of configurable counters (9), which are each coupled for signalling to at least one comparator (19) of the respective pixel element (5) of the partial number of the large number of pixel elements and to at least one comparator (19) of the at least one further pixel element (5) of the large number of pixel elements.

8. Photon-counting X-ray detector (1) according to one of claims 1 to 7, wherein the coincidence count signal is based on the signal that has been received directly in the respective pixel element (5) of the partial number of the large number of pixel elements and on coincident signals of between one further pixel element (5) and 24 further pixel elements (5) of the large number of pixel elements.

9. Photon-counting X-ray detector (1) according to one of claims 1 to 8, wherein the number and/or choice of further pixel elements (5) of the large number of pixel elements on which the coincidence count signal is based is different for different pixel elements (5).

10. Photon-counting X-ray detector (1) according to one of claims 1 to 9, wherein the number and/or choice of further pixel elements (5) of the large number of pixel elements, on which the coincidence count signal for a pixel element (5) arranged at the edge within the matrix of pixel elements (5) is based, is different from the number and/or choice of further pixel elements (5) of the large number of pixel elements, on which the coincidence count signal for a pixel element arranged centrally within the matrix of pixel elements (5) is based.

11. Photon-counting X-ray detector (1) according to one of claims 1 to 10, wherein each pixel element (5) of the partial number of the large number of pixel elements also comprises at least one electronic element (25) for preventing paralysis of the at least one configurable counter (9).

12. Medical imaging device having a photon-counting X-ray detector (1) according to one of the preceding claims.

13. Medical imaging device according to claim 12, wherein the medical imaging device is designed as a computed tomography system.

14. Method for operating a photon-counting X-ray detector (1), which is designed according to one of claims 1 to 11, for acquiring an X-ray image data set of an object (39) penetrated by X-ray radiation, wherein in a first counting mode the at least one configurable counter (9) of a pixel element (5) of the partial number of the large number of pixel elements (5) counts a pixel count signal, which is based on a signal that has been received directly in the pixel element (5) of the partial number of the large number of pixel elements (5) and in a second counting mode of the configurable counter (9) of the pixel element (5) counts a coincidence count signal, which is based on the signal that has been received directly in the respective pixel element (5) and on a coincident signal of at least one further pixel element (5) of the large number of pixel elements, and wherein it is possible to optionally switch between the first and the second counting modes.

## Revendications

1. Détecteur (1) de rayons X à comptage de photons pour l'enregistrement d'un ensemble de données d'image de rayons X d'un objet (39) traversé par un rayonnement X, comportant un élément (3) convertisseur, constitué pour transformer du rayonnement X incident en un signal électrique, et une matrice ayant une pluralité d'éléments (5) de pixels, dans lequel
- au moins un nombre partiel de la pluralité d'éléments de pixels a une entrée (7) de signal et au moins un compteur (9) configurable couplé en technique du signal avec celle-ci, et
- le compteur (9) configurable est constitué au choix, soit pour compter un signal de comptage de pixel, qui repose sur un signal entré directement dans chaque élément (5) de pixel du nombre partiel de la pluralité d'éléments de pixels, soit pour compter un signal de comptage de coïncidence, qui repose sur le signal entré directement dans l'élément (9) de pixel respectif et sur un signal apparaissant en coïncidence d'au moins un autre élément (5) de pixel de la pluralité d'éléments de pixels,
**caractérisé en ce que** le au moins un compteur (9) configurable a un multiplexeur (11) configurable monté en amont d'un élément (13) de comptage, qui a au moins un premier et un deuxième réglages, dans lequel dans le premier réglage du multiplexeur (11), le signal de comptage de pixel est compté au moyen de l'élément (13) de comptage et est mis en mémoire, et dans lequel dans le deuxième réglage du multiplexeur (11) le signal de comptage de coïncidence est compté et mis en mémoire au moyen de l'élément (13) de comptage.

2. Détecteur (1) de rayons X à comptage de photons suivant la revendication 1, dans lequel le au moins un compteur (9) configurable est configurable pour chaque élément (5) de pixel du nombre partiel de la pluralité d'éléments (5) de pixels individuellement ou respectivement pour un groupe d'éléments (5) de pixels du nombre partiel de la pluralité d'éléments (5) de pixels conjointement.

3. Détecteur (1) de rayons X à comptage de photons suivant l'une des revendications 1 ou 2, dans lequel chaque élément (5) de pixel de la pluralité d'éléments de pixels a un dispositif (15) de transformation relié à l'entrée du signal et ayant au moins un amplificateur (17) de signal et un nombre de comparateurs (19) ayant respectivement une valeur (THR) de seuil réglable, et dans lequel, pour chaque élément (5) de pixel du nombre partiel de la pluralité d'éléments de pixels, au moins un comparateur (19) du nombre de comparateurs (19) est couplé en technique du signal avec le au moins un compteur (9) configurable.

4. Détecteur (1) de rayons X à comptage de photons suivant la revendication 3, dans lequel chaque élément (5) de pixel du nombre partiel de la pluralité d'éléments de pixels a en outre au moins une logique (21) de coïncidence, qui est couplée en technique du signal avec au moins un comparateur (19) de l'élément (5) de pixel respectif du nombre partiel de la pluralité d'éléments de pixels et avec au moins un comparateur (19) du au moins un autre élément (5) de pixel de la pluralité d'éléments de pixels, dans lequel le signal de comptage de coïncidence repose sur un signal de sortie de la logique (21) de coïncidence.

5. Détecteur (1) de rayons X à comptage de photons suivant la revendication 4, dans lequel chaque élément (5) de pixel de la pluralité d'éléments de pixels a une pluralité de comparateurs (19), et dans lequel la au moins une logique (21) de coïncidence d'un élément (5) de pixel du nombre partiel de la pluralité d'éléments de pixels est couplée en technique du signal avec plus qu'un comparateur (19) du au moins un autre élément (5) de pixel de la pluralité d'éléments de pixels.

6. Détecteur (1) de rayons X à comptage de photons suivant la revendication 4 ou 5, dans lequel chaque élément (5) de pixel du nombre partiel de la pluralité d'éléments de pixels a au moins un élément (23) de réglage pour l'adaptation du temps de fonctionnement ou pour retarder un signal d'entrée dans la au moins une logique (21) de coïncidence.

7. Détecteur (1) de rayons X à comptage de photons suivant l'une des revendications 3 à 6, dans lequel chaque élément (5) de pixel du nombre partiel de la pluralité d'éléments (5) de pixels a plusieurs compteurs (9) configurables, qui sont couplés respectivement en technique de signal avec au moins un comparateur (19) de l'élément (5) de pixel respectif du nombre partiel de la pluralité d'éléments de pixels et avec au moins un comparateur (19) du au moins un autre élément (5) de pixel de la pluralité d'éléments de pixels.

8. Détecteur (1) de rayons X à comptage de photons suivant l'une des revendications 1 à 7, dans lequel le signal de comptage de coïncidence repose sur le signal entré directement dans l'élément (5) de pixel respectif du nombre partiel de la pluralité d'éléments de pixels et sur des signaux apparaissant en coïncidence entre un autre élément (5) de pixel et 24 autres éléments (5) de pixels de la pluralité d'éléments de pixels.

9. Détecteur (1) de rayons X à comptage de photons suivant l'une des revendications 1 à 8, dans lequel le nombre et/ou le choix d'autres éléments (5) de pixels de la pluralité d'éléments de pixels, sur lequel le signal de comptage de coïncidence repose, est différent pour des éléments (5) de pixels différents.

10. Détecteur (1) de rayons X à comptage de photons suivant l'une des revendications 1 à 9, dans lequel le nombre et/ou le choix d'autres éléments (5) de pixels de la pluralité d'éléments de pixels, sur lequel repose le signal de comptage de coïncidence pour un élément (5) de pixel disposé du côté du bord au sein de la matrice d'éléments (5) de pixels, est différent du nombre et/ou du choix d'autres éléments (5) de pixels de la pluralité d'éléments de pixels, sur lequel repose le signal de comptage de coïncidence pour un élément de pixel disposé au milieu au sein de la matrice d'éléments (5) de pixels.

11. Détecteur (1) de rayons X à comptage de photons suivant l'une des revendications 1 à 10, dans lequel chaque élément (5) de pixel du nombre partiel de la pluralité d'éléments de pixels comprend en outre au moins un élément (25) électronique pour empêcher une paralysie du au moins un compteur (9) configurable.

12. Appareil d'imagerie médicale comportant un détecteur (1) de rayons X à comptage de photons suivant l'une des revendications précédentes.

13. Appareil d'imagerie médicale suivant la revendication 12, dans lequel l'appareil d'imagerie médicale est constitué sous la forme d'un système de tomodensitométrie assisté par ordinateur.

14. Procédé pour faire fonctionner un détecteur (1) de rayons X à comptage de photons, qui est réalisé suivant l'une des revendications 1 à 11 pour l'enregistrement d'un ensemble de données d'image de rayons X d'un objet (39) traversé par un rayonnement X, dans lequel dans un premier mode de comptage, le au moins un compteur (9) configurable compte un élément (5) de pixel du nombre partiel de la pluralité d'éléments (5) de pixels, qui repose sur un signal entré directement dans l'élément (5) de pixel du nombre partiel de la pluralité d'éléments (5) de pixels, et dans un deuxième mode de comptage, le compteur (9) configurable de l'élément (5) de pixel compte un signal de comptage de coïncidence, qui repose sur le signal entré directement dans l'élément (5) de pixel respectif et sur un signal apparaissant en coïncidence d'au moins un autre élément (5) de pixel de la pluralité d'éléments de pixels, et dans lequel on peut commuter au choix entre le premier et le deuxième modes de comptage.
